(19) Europäisches Patentamt
European Patent Office
Office européen des brevets

(11) Publication number: **0 140 709 B1**

# (12) EUROPEAN PATENT SPECIFICATION

(45) Date of publication of patent specification: **03.04.91**

(21) Application number: **84307480.8**

(22) Date of filing: **30.10.84**

(51) Int. Cl.[5]: **A61K 31/54**, A61K 31/535, A61K 31/495, C07D 513/04, //C07D498/04,C07D513/16, C07D241/18,(C07D513/04, 279:00,241:00)

## (54) 5-Lipoxygenase inhibitors.

(30) Priority: **31.10.83 US 547161**

(43) Date of publication of application:
**08.05.85 Bulletin 85/19**

(45) Publication of the grant of the patent:
**03.04.91 Bulletin 91/14**

(84) Designated Contracting States:
**CH DE FR GB IT LI NL**

(56) References cited:
**EP-A- 0 085 881**

**JOURNAL OF MEDICINAL CHEMISTRY, vol. 26, no. 4, April 1983, pages 564-569, American Chemical Society, Washington, US; W.S. SAARI et al.: "Preparation of some 10-[3-(dimethylamino)-1-propyl]-10H-pyrazino[2,3-b][1,4]benzothiazines as potential neuroleptics"**

(73) Proprietor: **MERCK FROSST CANADA INC.
16711 Trans-Canada Highway
Kirkland Quebec(CA)**

(72) Inventor: **Atkinson, Joseph G.
5643 Plantagenet St.
Montreal Quebec, H3T 1S3(CA)**
Inventor: **Belanger, Patrice C.
419 Promenade d'Avignon
Dollard Des Ormeaux Quebec, H9B 1Y4(CA)**
Inventor: **Guindon, Yvan
409 Place Closse Ile Bizard
Quebec, H9C 1Y7(CA)**
Inventor: **Rokach, Joshua
416 Place Canterbury
Chomedey Laval Quebec, H7W 4G9(CA)**

(74) Representative: **Crampton, Keith John Allen et al
D. YOUNG & CO. 10 Staple Inn
London WC1V 7RD(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention is directed to inhibitors of the 5-lipoxygenase enzyme system of the arachidonic acid cascade. Inhibition of 5-lipoxygenase prevents the biosynthesis of the leukotrienes.

The leukotrienes are a novel group of biologically active mediators derived from arachidonic acid through the action of lipoxygenase enzyme systems. There are two groups of leukotrienes derived from the common unstable precursor Leukotriene $A_4$. The first of these are the peptido-lipid leukotrienes, the most important being Leukotrienes $C_4$ and $D_4$. These compounds collectively account for the biologically active material known as the slow reacting substance of anaphylaxis.

The leukotrienes are potent smooth muscle contracting agents, particularly on respiratory smooth muscle but also on other tissues (e.g. gall bladder). In addition, they promote mucous production, modulate vascular permeability changes and are potent inflammatory agents in human skin. The most important compound in the second group of leukotrienes is Leukotriene $B_4$, a dihydroxy fatty acid. This compound is a potent chemotactic agent for neutrophils and eosinophils and in addition, may modulate a number of other functions of these cells. It also effects other cell types such as lymphocytes and for example may modulate the action of T-suppressor cells and natural killer cells. When injected in vivo , in addition to promoting the accumulation of leukocytes, Leukotriene $B_4$ is also a potent hyperalgesic agent and can modulate vascular permeability changes through a neutrophil dependent mechanism. Both groups of leukotrienes are formed following oxygenation of arachidonic acid through the action of a 5-lipoxygenase enzyme. See for example, D. M. Bailey et al ., Ann. Rpts. Med. Chem. 17 203 (1982).

The leukotrienes are potent spasmogens of human trachea, bronchus and lung parenchymal strips, and when administered to normal volunteers as aerosols are 3,800 times more potent that histamine at inducing a 50% decrease in air flow at 30% of vital capacity. They mediate increases in vascular permeability in animals and promote mucous production in human bronchial explants. In addition, Leukotriene $B_4$ may also mediate mucous production and could be an important mediator of neutrophil and eosinophil accumulation in asthamatic lungs. 5-Lipoxygenase products are also thought to be regulators of mast cell degranulation and recent studies with human lung mast cells have suggested that 5-lipoxygenase inhibitors, but not corticosteroids, may suppress antigen-induced mast cell degranulation. In vitro studies have shown that antigen challenge of human lung results in the release of leukotrienes and in addition purified human mast cells can produce substantial amount of leukotrienes. There is therefore good evidence that leukotrienes are important mediators of human asthma. 5-Lipoxygenase inhibitors would therefore be a new class of drugs for the treatment of asthma.

Psoriasis is a human skin disease which effects between two and six percent of the population. There is no adequate therapy for psoriasis and related skin conditions. The evidence for leukotriene involvement in these diseases is as follows. One of the earliest events in the development of prepapillary lesions is the recruitment of leukocytes to the skin site. Injection of Leukotriene $B_4$ into human skin results in a pronounced neutrophil accumulation. There are gross abnormalities in arachidonic acid metabolism in human psoriatic skin. In particular, highly elevated levels of free arachidonic acid can be measured as well as large amounts of lipoxygenase products. Leukotriene $B_4$ has been detected in psoriatic lesions, but not in uninvolved skin, in biologically significant amounts.

Leukotrienes can be measured in nasal washings from patients with allergic rhinitis and are greatly elevated following antigen challenge. Leukotrienes may mediate this disease through their ability to regulate mast cell degranulation, by modulating mucous production and mucocillary clearance and by mediating the accumulation of inflammatory leukocytes.

Leukotrienes can also mediate other diseases. These include atopic dermatitis, gouty arthritis and gall bladder spasms. In addition, they may have a role in cardiovascular disease because leukotrienes $C_4$ and $D_4$ act as coronary and cerebral arterial vasoconstrictors and these compounds may also have negative inotropic effects on the myocardium. In addition, the leukotrienes are important mediators of inflammatory diseases through their ability to modulate leukocyte and lymphocyte function. See, for example, B. Samuelson, Science , 220 , 568 (1983).

Several compounds having the Formula A (especially wherein Z=S) are taught in the literature as pesticides and herbicides:

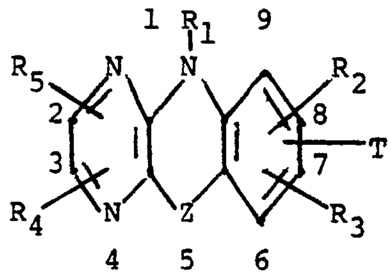

A

see for example: S.D. Carter et al ., Tetrahedron 33 827 (1977); G.W.H. Cheeseman et al ., Tetrahedron 36 2681 (1980); C.O. Okafor, J. Het. Chem. 18 405-407 and 1445-1449 (1981); and U.S. Patent Nos. 3.663,543; 3,746,707; 3,808,208; 3,821,213 and 3,845,044. None of the compounds of Formula A is taught to have 5-lipoxygenase inhibiting properties.

European Patent Specification EP-A-0 085 881 discloses certain pyrazolooxazines, thiazines, and quinolines, and their use in medicine, especially as lipoxygenase inhibitors.

J Med. Chem. (1983), 26 , 564-9 discloses studies of certain chloro-substituted and methyl-substituted 10-(3-dimethylamino-1-propyl)-10H -pyrazino[2,3-b][1,4]benzothiazines. In these studies, which involved receptor-binding assays, in vivo behavioural tests and electrochemical oxidation studies, none was found to be as effective as chlorpromazine in tests predictive of neuroleptic activity.

It has been discovered that compounds of the Formula A are effective inhibitors of leukotriene biosynthesis via inhibition of the mammalian 5-lipoxygenase enzyme system. Thus, these compounds are useful therapeutic agents for treating conditions such as asthma, allergies, cardiovascular disorders such as angina and inflammation, and skin diseases such as psoriasis.

The compounds of the present invention may also be used erosive gastritis; erosive esophagitis; inflammatory bowel disease; ethanol-induced hemorrhagic erosions; hepatic ischemia; noxious agent induced damage or necrosis of hepatic, pancreatic, renal, or myocardial tissue; liver parenchymal damage caused by hepatoxic agents such as $CCl_4$ and D-galactosamine; ischemic renal failure; disease-induced hepatic damage; bile salt induced pancreatic or gastric damage; trauma- or stress-induced cell damage; and glycerol-induced renal failure.

The present invention provides pharmaceutical compositions for inhibiting leukotriene biosythesis or action and comprising a compound of the Formula I:

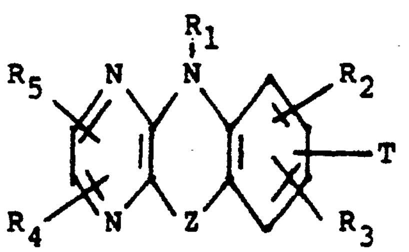

I

or a compound that is a pharmaceutically acceptable salt thereof, together with a pharmaceutically acceptable carrier, where, in the formula,

Z is O, NCN, S, SO or $SO_2$;

$R_1$ is H, $C_{1-6}$ alkyl, benzyl, $C_{1-6}$ acyl, $C_{1-6}$ aminoacyl, ($C_{1-6}$ alkylacyloxy)-($C_{1-6}$ alkyl), (for example, $-CH(CH_3)OCOC(CH_3)_3$), ($C_{1-6}$ alkoxy)-($C_{1-6}$ alkyl), (for example, $-CH(CH_3)OC_2H_5$), $-(CH_2)_nCOOR_6$ where n is 0, 1, 2, 3, or 4, CN, ($C_{1-6}$ alkyl)acyloxy-($C_{1-6}$ alkoxy)carbonyl, (e.g. $-COOCH(O_2CCH_3)CH_3$);

each of $R_2$, $R_3$, $R_4$ and $R_5$, independently of the others, is hydrogen, $C_{1-6}$ alkyl, $C_{2-6}$ alkenyl or $-(CH_2)_nM$, where n is 0 to 6 and

M is

(a) $OR_{16}$

(b) halogen;

(c) $CF_3$;

(d) $SR_{16}$;

(e) phenyl;

(f) substituted phenyl where the substituents(s) is/are $C_{1-3}$ alkyl, halogen, CN, $C_{1-3}$ alkoxy, OH, $(CH_2)_nNR_8R_9$ where n is 0, 1 or 2, $CF_3$, $COOR_6$ or $CH_2COOR_6$; (herein called "substituted phenyl as defined");
(g) $COOR_6$;
(h) $-CO-R_{14}$;
(i) tetrazolyl;
(j) $NH-CO-R_7$;
(k) $NR_8R_9$;
(l) $NHSO_2R_{10}$;
(m) $-CO-CH_2OH$;
(n) $SOR_{11}$, where $R_{11}$ is $C_{1-6}$ alkyl, phenyl, substituted phenyl as defined, $(CH_2)_mCOOR_6$, where m is an integer from 1 to 6, or $CF_3$;
(o) $CONR_8R_9$;
(p) $SO_2NR_8R_9$;
(q) $SO_2R_{13}$ where $R_{13}$ is OH, $C_{1-6}$ alkyl, H, phenyl, substituted phenyl as defined, $(CH_2)_mCOOR_6$ or $CF_3$;
(r) $NO_2$;
(s) $-OCO-R_{14}$;
(t) $-OCO-NR_8R_9$;
(u) $-OCO-OR_7$; or
(v) -CN;

each $R_{16}$, independently of any other, is H; ($C_{1-6}$ alkoxy)-($C_{1-6}$ alkyl); ($C_{1-6}$ alkyl)acyloxy-($C_{1-6}$ alkyl); $C_{1-6}$ alkyl; substituted phenyl as defined; $-(CH_2)_mCOOR_6$ in which m is 0 to 6; CN: $C_{1-5}$ alkylacyl; $C_{1-4}$ perfluoroalkyl; phenyl; benzyl; or $CH_2-R_{12}$ where $R_{12}$ is $C_{1-5}$ alkyldimethylamino;
each $R_6$, independently of any other, is H, $C_{1-6}$ alkyl, benzyl or phenyl;
each $R_{14}$, independently of any other, is H, $C_{1-6}$ alkyl, $C_{1-6}$ alkoxy, $C_{1-6}$ alkyl)acyloxy-($C_{1-6}$ alkoxy), $(CH_2)_nCOOR_6$ where n is 0 to 4, phenyl or substituted phenyl as defined or is such that $R_{14}COOH$ is an essential amino acid;
each of $R_8$ and $R_9$, independently of the others, is H, phenyl, substituted phenyl as defined or $C_{1-4}$ alkyl, or $NR_8R_9$ is a heterocycloalkyl of 5 to 8 ring atoms; and
each $R_7$, independently of the others, is H, $C_{1-6}$ alkyl, benzyl, phenyl or ($C_{1-6}$ alkyl)acyloxy-($C_{1-6}$ alkoxy);
each $R_{10}$ is independently OH, $C_{1-6}$ alkyl, $C_{1-6}$ alkoxy, phenyl or p-tolyl;
or any two of $R_1$, $R_2$, $R_3$, $R_4$ or $R_5$ are joined to form an additional ring of 5 to 7 members that optionally contains a carbonyl group and/or a hydroxyl group as substituent(s), and that has 0, 1 or 2 double bonds, and such that, if $R_1$ is a constituent of the ring, one member is nitrogen and the others are carbon and if $R_1$ is not a constituent of the ring, all the members are carbon; and
T is hydrogen or $OR_{15}$ where $R_{15}$ is hydrogen, $C_{1-6}$ alkyl, $C_{1-6}$ alkylacyl, phenyl-($C_{1-8}$ alkyl)acyl, $SO_2R_{10}$, arylsulfonyl, -CO-phenyl or substituted phenyl as defined.

In a preferred embodiment, Z is O, S, SO or $SO_2$ and n is 0 or 1, especially 0, in the unit $(CH_2)_nM$, the remaining substituents being as defined for Formula I.

In another preferred embodiment,
Z is O or S;
$R_1$ is H, $C_{1-6}$ alkyl, $C_{1-6}$ acyl, $-(CH_2)_nCOOR_6$, ($C_{1-6}$ alkyl)acyloxy -($C_{1-6}$ alkoxy)carbonyl, (e.g. $-COOCH(O_2CCH_3)CH_3$), $-C(H)=C(H)COOR_6$ or $SO_2R_{10}$;
each of $R_2$, $R_3$, $R_4$ and $R_5$, independently of the others, is hydrogen, $C_{1-6}$ alkyl, or $-(CH_2)_nM$, wherein:
n is 0 or an integer from 1 to 6 and
M is
(a) $OR_{16}$
(b) halogen;
(c) $CF_3$;
(d) $NO_2$;
(e) $-OCO-R_{14}$;
(f) $-OCO-NR_8R_9$; or
(g) $-OCO-OR_7$; and the other variables are as defined above, but with the proviso that there is a group $OR_{16}$ located at one of the 6, 7, 8 and 9-positions.

The term alkyl, as used herein, includes straight chain, branched chain and cyclic groups.

The term aryl, as used herein, includes phenyl, naphthyl, anthracenyl, and the like.

The term heteroaryl includes 5- and 6-membered rings containing at least one heteroatom selected from N, O, S, for example, furyl, thienyl, pyrrolyl, pyridyl.

The term essential amino acid is employed to include the following amino acids; lysine, tryptophan, histidine, phenylalanine, leucine, isoleucine, threonine, methionine, valine, arginine, alanine, proline, glycine, serine, cysteine, tyrosine, asparagine, glutamine, aspartic acid and glutamic acid.

Whenever possible, appropriate pharmaceutically acceptable salts of Formula I are included within the definitions given above. These include carboxylic or mineral acid addition salts where Formula I is basic and salts of pharmaceutically acceptable bases when Formula I is acidic. Salts derived from inorganic bases include sodium, potassium, lithium, ammonium, calcium, magnesium, ferrous, zinc, copper, manganous, aluminum, ferric and manganic salts. Particularly preferred are the ammonium, potassium, sodium, calcium and magnesium salts. Salts derived from pharmaceutically acceptable organic non-toxic bases include salts of primary, secondary and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, trimethamine, lysine, arginine, histidine, caffeine, procaine, hydrabamine, choline, betaine, ethylenediamine, glucosamine, methylglucamine, theobromine, purines, piperazine, piperidine, N-ethylpiperidine and polyamine resins. Particularly preferred organic non-toxic bases are isopropylamine, diethylamine, ethanolamine, piperidine, trimethamine, choline and caffeine.

When the compound is basic, salts may be prepared from pharmaceutically acceptable non-toxic acids, including inorganic and organic acids. Such acids include hydrochloric, hydrobromic, sulfuric, nitric, isethionic, methanesulfonic, ethanesulfonic, benzenesulfonic, p-tolueuesulfonic, acetic, benzoic, comphorsulfonic, citric, fumaric, gluconic, glutamic, lactic, malic, maleic, mandelic, mucic, pamoic, pantothenic, phosphoric, succinic and tartaric acids. the like. Particularly preferred are hydrochloric, hydrobromic, citric, maleic, phosphoric, sulfuric and tartaric acids.

For a helpful discussion of pharmaceutical salts see S.M. Berge et al ., Journal of Pharmaceutical Sciences, 66 , 1-19 (l977).

The compounds of the present invention are prepared by a series of reactions as illustrated in Scheme I. An initial condensation reaction between a vicinal dihalopyrazine (III) with an appropriately substituted aminobenzenethiol (IV) affords the compound (V).

Compound (V) is cyclized in the presence of a base, such as triethylamine and an inert solvent such as dimethylformamide (DMF) to afford the compound of Formula I wherein Z = S.

Compounds of Formula I wherein Z = S may be oxidized by contacting the Formula I compounds with 30-35% hydrogen peroxide in an acidic solvent for several hours at a temperature of from about 75° to 80° C. In some cases, higher peroxide concentrations, higher temperatures and/or more prolonged contact times may be required, especially when the sulfone is the desired product. Acetic acid is generally a useful solvent, but other acids, such as trifluoroacetic acid, can also be employed.

Similarly, the compounds of Formula I wherein Z = O or NCN may be produced by following Scheme I, but substituting the appropriate compound corresponding to Formula (IV) for the aminobenzenethiol shown.

It will be obvious to the skilled artisan that the substituents present in the compounds of formulae III, IV and V must be such that they are compatible with the reaction conditions used in the preparation of Formula I.

SCHEME I

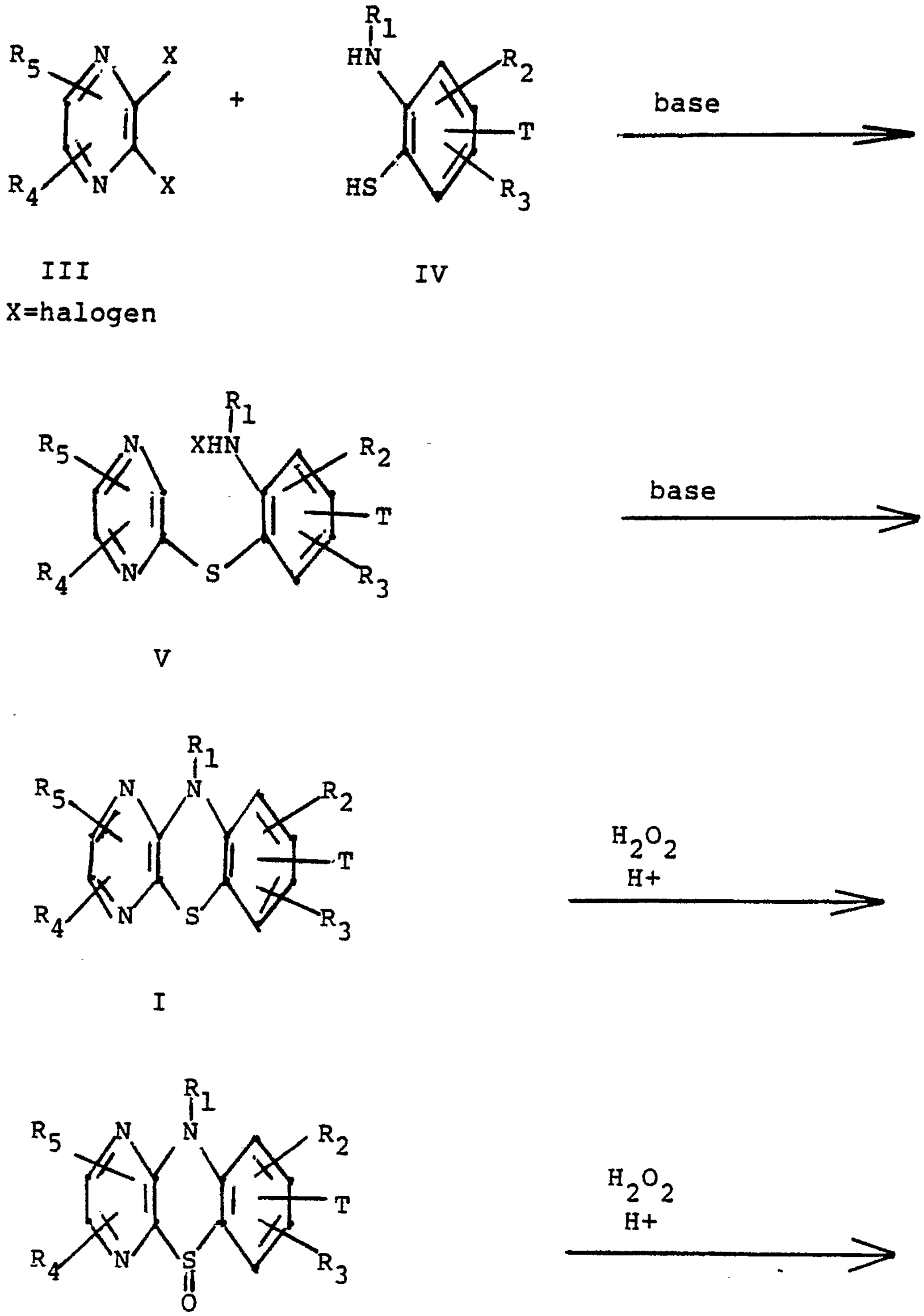


III

X=halogen

IV

V

I

SCHEME I (cont'd)

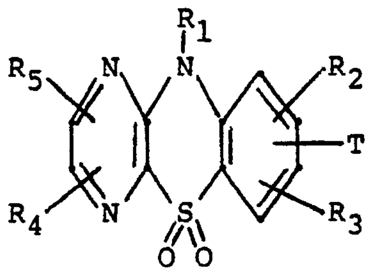

An alternative procedure for preparing compounds of Formula I substituted at position 10 is illustrated in Scheme II. Thus, an alkylating agent is reacted with a compound of Formula VI in the presence of a base in an inert solvent to yield compounds of Formula I. A side product of Formula VII, resulting from alkylation at position I is sometimes obtained in this reaction.

SCHEME II

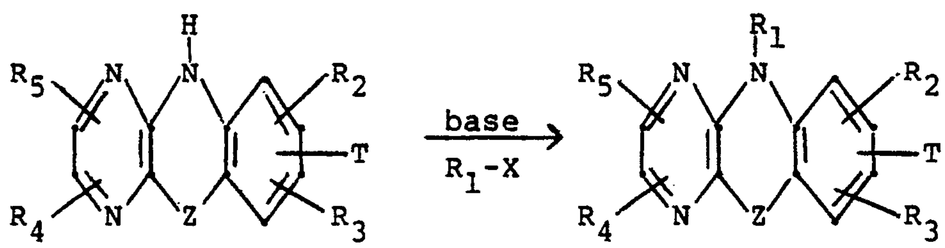


VI I

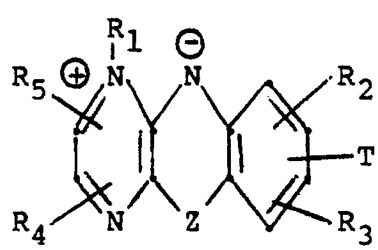

VII

The compounds of the Formula I have unexpected activity as inhibitors of the mammalian biosynthesis of leukotriene $B_4$, as well as leukotrienes $C_4$, $D_4$, $E_4$ and $F_4$, the active elements of the slow reacting substance of anaphylaxis (SRS-A). The compounds of Formula I act as inhibitors of the mammalian 5-lipoxygenase enzyme system of the arachidonic acid cascade. This inhibition of the mammalian biosynthesis of leukotrienes indicates that the compositions are useful to treat, prevent or ameliorate, in mammals and especially in humans 1) pulmonary conditions including diseases such as asthma, 2) allergies and allergic reactions such as allergic rhinitis, contact dermatitis, allergic conjunctivitis and the like, 3) inflammation such as arthritis, 4) pain, 5) skin conditions such as psoriasis and the like and 6) cardiovascular conditions such as angina and the like.

Examples of the Formula I compounds useful in the present compositions are tabulated in Table I. The number preceding the $R_2$-$R_5$ and T definitions signifies that group's position on the ring system.

TABLE I

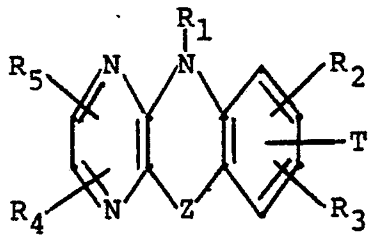

| Compound | Z | $R_1$ | $R_2$ | $R_3$ | $R_4$ | $R_5$ | T |
|---|---|---|---|---|---|---|---|
| 1 | S | H | H | H | H | H | H |
| 2 | S | $CH_3$ | H | H | H | H | H |
| 3 | S | $CH_2Ph$ | H | H | H | H | H |
| 4 | S | H | 3-$NO_2$ | H | H | H | H |
| 5 | S | H | 2-Cl | 3-Cl | H | H | H |
| 6 | S | H | 3-$NO_2$ | 7-$NO_2$ | H | H | H |
| 7 | S | $CH_3$ | 2-$NO_2$ | H | H | H | H |
| 8 | S | $CH_3$ | 3-$NO_2$ | H | H | H | H |
| 9 | S | n-$C_3H_7$ | 2-Cl | 3-Cl | H | H | H |
| 10 | S | n-$C_6H_{13}$ | 2-Cl | 3-Cl | H | H | H |
| 11 | S | $CH_2Ph$ | 2-Cl | 3-Cl | H | H | H |
| 12 | SO | H | H | H | H | H | H |
| 13 | SO | $CH_3$ | H | H | H | H | H |
| 14 | SO | H | 2-Cl | 3-Cl | H | H | H |
| 15 | SO | H | 3-$NO_2$ | 7-$NO_2$ | H | H | H |
| 16 | $SO_2$ | $CH_3$ | H | H | H | H | H |
| 17 | $SO_2$ | $CH_2Ph$ | H | H | H | H | H |
| 18 | $SO_2$ | $CH_2Ph$ | 2-Cl | 3-Cl | H | H | H |
| 19 | O | H | H | H | H | H | H |
| 20 | O | H | 2-F | 3-F | H | H | H |
| 21 | O | H | 2-Cl | 3-Cl | H | H | H |
| 22 | O | H | 2-Br | 3-Br | H | H | H |
| 23 | O | H | 2-Cl | 3-Cl | 8-$CH_3$ | H | H |
| 24 | N-CN | H | H | H | H | H | H |

| | | | | | | | | |
|---|---|---|---|---|---|---|---|---|
| 25 | N-CN | $CH_3$ | 2-$CH_3$ | 3-$CH_3$ | H | H | H |
| 26 | N-CN | $CH_3$ | H | H | H | H | 7-$OCH_3$ |
| 27 | S | H | 2-Cl | H | H | H | H |
| 28 | S | H | 3-Cl | H | H | H | H |
| 29 | S | H | 8-Cl | H | H | H | H |
| 30 | S | H | 2-$CH_3$ | H | H | H | H |
| 31 | S | H | H | H | H | H | 7-$OCH_3$ |
| 32 | S | H | H | H | H | 8-Cl | 7-$OCH_3$ |
| 33 | S | $CH_3$ | H | H | H | 8-Cl | 7-$OCH_3$ |
| 34 | S | $CH_3$ | H | H | H | 8-Cl | 7-OH |
| 35 | S | $CH_3$ | H | H | H | H | 7-$OCH_3$ |
| 36 | S | H | H | H | H | H | 7-OH |
| 37 | S | $CH_3$ | H | H | H | H | 7-OH |
| 38 | SO | H | H | H | H | H | 7-$OCH_3$ |
| 39 | SO | $CH_3$ | H | H | H | H | 7-$OCH_3$ |
| 40 | S | $SO_2$Ph-p-Me | H | H | H | H | 7-$OCH_3$ |
| 41 | $SO_2$ | H | H | H | H | H | 7-$OCH_3$ |
| 42 | S | $COCH_3$ | H | H | H | H | 7-$OCH_3$ |
| 43 | S | $COCH_3$ | H | H | H | H | H |
| 44 | $SO_2$ | H | H | H | H | H | H |
| 45 | S | $CH_2CO_2C_2H_5$ | H | H | H | H | H |
| 46 | S | $CH_2CO_2H$ | H | H | H | H | H |
| 47 | S | $CO_2CH_3$ | H | H | H | H | H |
| 48 | S | $CH=CHCO_2CH_3$ | H | H | H | H | H |

Representative compounds of Formula I have been tested using one or more of the following assays to determine their mammalian leukotriene biosynthesis inhibiting activity and other relevant biological activities.

Rat Polymorphonuclear Leukocyte

(P.M.N.) Assay

Rats under ether anesthesia were injected (i.p.) with 8 ml of a suspension of sodium caseinate (6 grams in ca . 50 ml water). After 15-24 hours the rats were sacrificed ($CO_2$) and the cells from the peritoneal cavity were recovered by lavage with 20 ml of buffer (Eagles MEM containing 30 mM HEPES adjusted to pH 7.4 with NaOH). The cells were pelleted (350 x g, 5 min.), resuspended in buffer with vigorous shaking, filtered through lens paper, recentrifuged and finally suspended in buffer at a concentration of 10 cells/ml. A 500 µl aliquot of PMN suspension and test compound were preincubated for 2 minutes at 37° C, followed by the addition of 10 µM A-23187. The suspension was stirred for an additional 4 minutes then bioassayed for $LTB_4$ content by adding an aliquot to a second 500 µl portion of the PMN at 37° C. The $LTB_4$ produced in the first incubation caused aggregation of the second PMN, which was measured as a change in light transmission. The size of the assay aliquot was chosen to give a submaximal transmission change (usually -70%) for the untreated control. The percentage inhibition of $LTB_4$ formation was calculated from the ratio of

transmission change in the sample to the transmission change in the compound-free control.

Antigen Challenge '*in vitro*' Assay

Male guinea pigs weighing 300-350 g were sensitized by injecting (I.P.) 0.5 ml of a suspension containing 0.4 mg of egg albumin (Ovalbumin, Grade V, Sigma Chemical Co.) and 4.0 g aluminum hydroxide in 19.6 ml of saline. Two weeks were permitted for sensitization to occur.

Three sensitized guinea pigs were stunned and exsanguinated. The tracheas were removed, freed of adhering tissue and divided longitudinally by cutting through the cartilaginous tissue directly opposite the muscle insertion. Each opened trachea was then transected between every second cartilage. Four of the cut sections were tied together, end to end, in a series with No.7 silk thread ensuring that the tracheal muscles were all in the same vertical plane. Thus, each chain consisted of tissue from three different animals.

The chain so formed was then suspended under 1 g of tension (by silk ties at each end) in a 20 ml organ bath containing 10 ml of modified [1]Krebs-Henseleit buffer solution gassed with 95% $O_2$ and 5% $CO_2$ at 37° C. Mepyramine (0.55 μg/ml) and indomethacin (2.67 μg/ml) were added to the buffer to avoid the contribution of histamine receptors and cyclooxygenase products to the contraction. To record responses one end of the tracheal chain was attached to a Gould-Statham UC-2 force displacement transducer which was connected to a Beckman Type R-dynograph. The preparations were allowed to equilibrate for one hour during which time the tissues were automatically washed (10 ml volume displacement) every 6 minutes.

After the equilibration period the tissues were primed with methacholine (3 μg/ml; 1.5 x $10^{-5}$M), washed and allowed to recover to baseline. The tissues were treated again with a second dose of methacholine, washed, allowed to return to baseline and washed for an additional hour.

Two chains were used as a control. These were incubated in a concentration of egg albumin sufficient to induce an average contraction of 50-80% of the methacholine response.

Each compound to be tested was added to two other baths (at a final concentration in each bath of 10 μg/ml) 15 minutes prior to challenging the fresh chains with egg albumin.

The response of the challenged tissue was expressed as a percentage of the methacholine maximum. The percentage inhibition for each compound was then calculated. Compounds which at 10 μg/ml (final conc.) inhibited the egg albumin response by 50% or more were retested at a lower concentration.

Asthmatic Rat Assay

Rats were obtained from an inbred line of asthmatic rats. Both female and male rats from 200 to 300 g were used.

Egg albumin (EA), grade V, crystallized and lyophilized, was obtained from Sigma Chemical Co., St. Louis. *Bordetella pertussis* vaccine, containing 30 x $10^9$ killed bacteria per ml was obtained from the Institut Armand-Frappier, Laval des Rapides, Quebec. Aluminum hydroxide was obtained from the Regis Chemical Company, Chicago.

The challenge and subsequent respiratory recordings were carried out in a clear plastic box with internal dimensions 10 x 6 x 4 inches. The top of the box was removable; in use, it was held firmly in place by four clamps and an airtight seal was maintained by a soft rubber gasket. Through the center of each end of the chamber a Devilbiss nebulizer (No. 40) was inserted via an airtight seal and each end of the box also had an outlet. A Fleisch No. 0000 pneumotachograph was inserted into one end of the box and coupled to a Grass volumetric pressure transducer (PT5-A) which was then connected to a Beckman Type R Dynograph through appropriate couplers. While aerosolizing the antigen, the outlets were open and the pneumotachograph was isolated from the chamber. The outlets were closed and the pneumotachograph and the chamber were connected during the recording or the respiratory patterns. For challenge, 2 ml of a 3% solution of antigen in saline was placed into each nebulizer and the aerosol was generated with air from a small Potter diaphragm pump operating at 10 psi and a flow of 8 liters/minute.

Rats were sensitized by injecting (s.c.)1 ml of a suspension containing 1 mg EA and 200 mg aluminum hydroxide in saline. Simultaneously, they received an intraperitoneal (i.p.) injection of 0.5 ml of *B. pertussis* vaccine. They were used between days 14 and 18 postsensitization. In order to eliminate the serotonin

[1]modified Krebs solution in grams/liter and (mM): NaCl - 6.87 (120); glucose - 2.1 (11); $NaHCO_3$ 2.1 (25); KCl - 0.32 (4.72); $CaCl_2$ - 0.28 (2.5); $MgSO_4$-$7H_2O$ - 0.11 (0.5); $KH_2PO_4$ - 0.16 (1.2); pH at bathing solution = 7.35 ± 0.05.

component of the response, rats were pretreated intravenously 5 minutes prior to aerosol challenge with 30 gm/kg methylserzide. Rats were then exposed to an aerosol of 3% EA in saline for exactly 1 minute, then their respiratory profiles were recorded for a further 25-30 minutes. The duration of continuous dyspnoea was measured from the respiratory recordings.

Compounds were generally administered either intraperitoneally 1 hour prior to challenge or orally 1-1/2 hours prior to challenge. They were either dissolved in dimethylsulfoxide or suspended in 0.1% methocel and 0.5% Tween 80. The volume injected was 2 ml/kg (intraperitoneally) or 10 ml/kg (orally (p.o.)). Prior to oral treatment rats were starved overnight. Their activity was determined in terms of their ability to decrease the duration of symptoms of dyspnoea in comparison with a group of vehicle-treated controls. Usually, a compound was evaluated at a series of doses and an $ED_{50}$ was determined. This was defined as the dose (mg/kg) which would inhibit the duration of symptoms by 50%.

PAF-Induced Hyperalgesia Assay

Female Sprague-Dawley rats, 35-40 g were fasted overnight. Platelet activating factor, PAF, (L-lecithin B-acetyl O-alkyl) 1 μg/0.1 ml was given by subplantar injection in the rat paw. The compounds to be evaluated were homogenized in Aqueous Vehicle (0.9% benzyl alcohol, 0.5% Tween 80 and 0.4% methylcellulose) and administered orally in a volume of 0.1 ml, 30 minutes prior to PAF.

Animals were tested 1, 2, 3 and 4 hours after PAF administration. The vocalization threshold, defined as the pressure (mmHg) needed to evoke a squeak response, was recorded for both the injected and contralateral paw. No animal was subjected to pressure greater than 60 mmHg. Hyperalgesia is defined as a decrease in vocalization threshold as compared to a normal paw. Percent inhibition of hyperalgesia was calculated as the proportion of animals with vocalization thresholds greater than 200% of controls.

Tables II and III below show data obtained using the above described assays with representative compounds of Formula I.

TABLE II

Assay Results

| Compound | PMN $I_{50}$ μg/ml | Antigen Challenge % Inhibition and μg/ml |
|---|---|---|
| 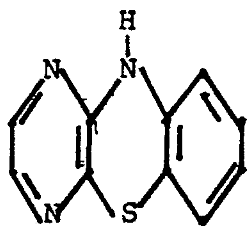 | 5 | 85% (10) |
| 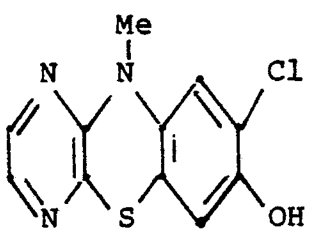 | 0.2 | |
| 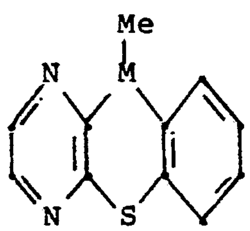 | 1-5 | - |
| 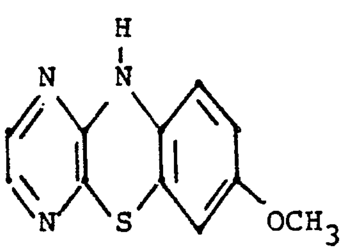 | 0.05-0.5 | 42% (3) |

TABLE II (cont'd)

| | | |
|---|---|---|
| 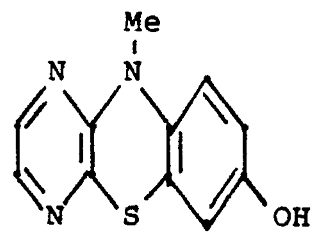 | 1-5 | 71% (3) |
| 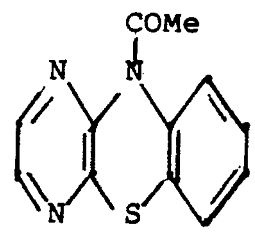 | - | 14% (3) |
| 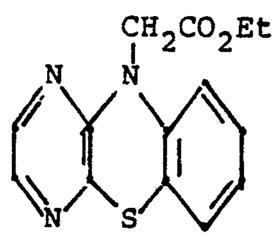 | - | 22% (3) |
| 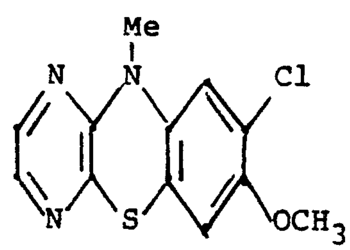 | 5 | - |
| 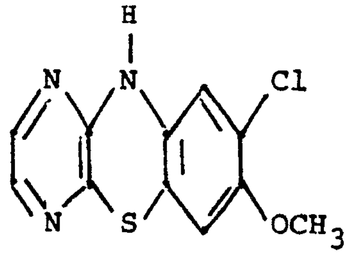 | 1 | - |

TABLE II (cont'd)

| | | |
|---|---|---|
| 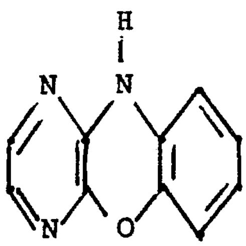 | 0.5-1 | 35% (3) |

TABLE III

| Compound | Asthmatic Rat % Inhibition, Dose | PAF % Inhibitiion, Dose |
|---|---|---|
| 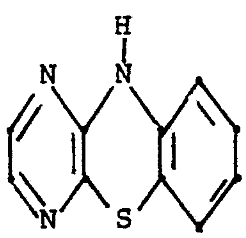 | 30% 5 mg/kg, p.o. | 70% 10 mg/kg, i.p. |
| 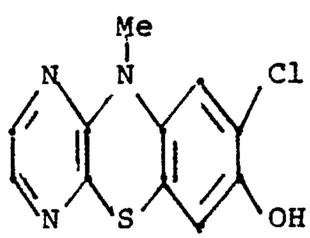 | 47% 5 mg/kg, p.o. | - |
| 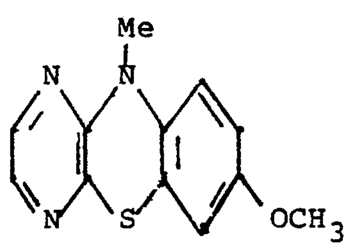 | 43% 3 mg/kg, p.o. | - |

TABLE III (cont'd)

| | | |
|---|---|---|
| 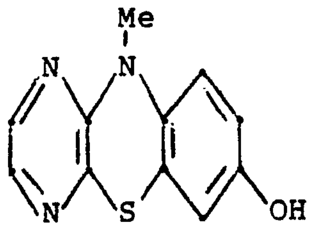 | 39% 3 mg/kg, p.o. | - |
| 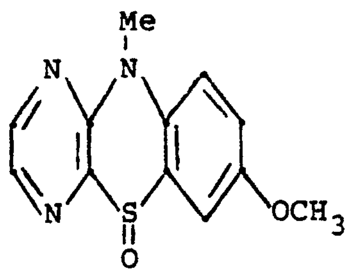 | 25% 3 mg/kg, p.o. | - |

The cytoprotective activity of a compound may be observed in both animal and man by noting the increased resistance of the gastrointestinal mucosa to the noxious effects of strong irritants, for example, the ulcerogenic effects of aspirin or indomethacin. In addition to lessening the effect of non-steroidal anti-inflammatory drugs on the gastrointestinal tract, animal studies show that cytoprotective compounds will prevent gastric lesions induced by oral administration of strong acids, strong bases, ethanol, hypertonic saline solutions and the like.

Two assays can be used to measure cytoprotective ability. These assays are; (A) an ethanol-induced lesion assay and (B) an indomethacin-induced ulcer assay.

A. Ethanol-Induced Gastric Ulcer Assay

Twenty-four hour fasted Sprague-Dawley (S.D.) rats are perorally (p.o.) dosed with 1.0 ml absolute ethanol. Fifteen to thirty minutes prior to ethanol administration, groups of rats each receive either an aqueous vehicle (aqueous methylcellulose 5% wt.) or the test compound at various doses perorally. One hour later, the animals are sacrificed and stomach mucosae are examined for resulting lesions.

B. Indomethacin-Induced Ulcer Assay

Indomethacin, 10 mg/kg p.o., is used to induce ulcers in 24 hour fasted S.D. rats. Fifteen minutes prior to indomethacin administration, groups of rats each receive either an aqueous vehicle (5% by weight methylcellulose) or the test compound at various doses perorally. Four hours later the animals are sacrificed and stomach mucosae are examined for resulting ulcers.

The magnitude of a prophylactic or therapeutic dose of a compound of Formula I will, of course, vary with the nature of the severity of the condition to be treated and with the particular compound of formula I and its route of administration. In general, the daily dose range for anti-asthmatic, anti-allergic or anti-inflammatory use and, generally, uses other than cytoprotection, lies within the range of from about 10 micrograms to about 20 mg per kg body weight of a mammal. This dosage may be administered in single or divided individual doses.

As cytoprotective agents, the leukotriene inhibitors of Formula I may generally be administered at a dosage range of 0.01 mg/kg to 20 mg/kg of body weight. The exact amount of inhibitor to be used will depend on, inter alia , whether it is being administered to heal damaged cells or to avoid future damage, on the nature of the damaged cells (e.g., gastro-intestinal ulcerations vs. nephrotic necrosis), and on the nature of the causative agent. An example of the use of a compound of the Formula I in avoiding future damage would be co-administration of a compound of the Formula I with a non-steroidal anti-inflammatory drug (NSAID) that might otherwise cause such damage (for example, idomethacin). For such use, the compound of Formula I is administered from 30 minutes prior up to 30 minutes after administration of the NSAID.

Preferably, it is administered prior to or simultaneously with the NSAID (for example, in a combination dosage form).

The effective daily dosage level for compounds of Formula I inducing cytoprotection in mammals, especially humans, will generally range from 0.01 mg/kg to 20 mg/kg, preferably from 0.01 mg/kg to 10 mg/kg. The dosage may be administered in single or divided individual doses.

Any suitable route of administration may be used for providing a mammal, especially a human, with an effective dosage of leukotriene inhibitor. For example, oral, rectal, transdermal, parenteral, intramuscular and intravenous administration are suitable. Dosage forms include tablets, troches, dispersions, suspensions, solutions and capsules.

The pharmaceutical compositions of the present invention comprise a compound of formula I as an active ingredient or a pharmaceutically acceptable acceptable salt thereof, and may also contain a pharmaceutically acceptable carrier and optionally other therapeutic ingredients. The compositions include compositions suitable for oral, rectal, ophthalmic, pulmonary, nasal, dermal, topical or parenteral (including subcutaneous, intramuscular and intravenous) administration, although the most suitable route in any given case will depend on the nature and severity of the conditions being treated and on the nature of the active ingredient. They may be conveniently presented in unit dosage form and prepared by any of the methods well known in the art of pharmacy.

For intravenous administration, a suitable dosage range for anti-asthmatic, anti-inflammatory or anti-allergic use is from 0.01 mg to 20 mg (preferably from 0.1 mg to 10 mg) of a compound of Formula I per kg of body weight per day and for cytoprotective use from 0.01 mg to 20 mg (preferably from 0.1 mg to 10 mg) of a compound of Formula I per kg of body weight per day. In the case where an oral composition is used, a suitable dosage range for anti-asthmatic, anti-inflammatory or anti-allergic use is, e.g. from 0.1 to 20 mg of a compound of Formula I per kg of body weight per day, preferably from 1 mg to 10 mg per kg, and for cytoprotective use from 0.1 mg to 20 mg (preferably from 0.1 mg to 10 mg) of a compound of Formula I per kg of body weight per day.

For treating pulmonary conditions such as asthma, the mode of administration may be, for example, oral, parenteral, by inhalation or by suppository. Suitable oral dosage forms are tablets, elixirs, emulsions, solutions and capsules, including delayed or sustained-release capsules. Parenteral dosage forms include solutions and emulsions. Dosage forms for administration by inhalation include sprays and aerosols. These inhalation formulations may be administered in metered doses ranging from 0.1 microgram to 200 micrograms, administered as needed.

For administration by inhalation, the compounds of the present invention are conveniently delivered in the form of an aerosol spray presentation from pressurized packs of a nebulizer. The preferred composition for inhalation is a powder which may be formulated as a cartridge from which the powder composition may be inhaled with the aid of a suitable device. In the case of a pressurized aerosol, the dosage unit may be determined by providing a valve to deliver a metered amount.

For treating allergies or allergic reactions, such as allergic conjunctivitis and allergic rhinitis, the Formula I compound may be administered by any conventional mode, e.g., orally, parenterally, topically, subcutaneously or by inhalation. The oral and parenteral dosage forms are of the same type as for the pulmonary treatment. The topical application dosage forms include ointments, salves, controlled release patches, emulsions, solutions, thixotropic formulations, powders and sprays. For topical application, the percent by weight of active ingredient (Formula I compound) may vary from 0.001 to 10%.

For treating inflammation the mode of administration may be oral, parenteral or by suppository. The various dosage forms are the same as those described above.

For treating skin diseases such as psoriasis and atopic dermatitis, oral, topical or parenteral administration is useful. For topical application to the diseased area, salves, patches, controlled-release patches and emulsions are convenient dosage forms.

For use as an analgesic, i.e. for treating pain, any suitable mode of administration may be used, e.g., oral, parenteral, by insufflation or by suppository.

For treating cardiovascular conditions such as angina pectoris, any suitable mode of administration, e.g. oral, parenteral, topical, or insufflation, and dosage from e.g. pills, liquid formulations, controlled-release capsules, or controlled-release skin patches may be used.

In practical use, leukotriene inhibitors of Formula I can be combined as the active ingredient in intimate admixture with a pharmaceutical carrier according to conventional pharmaceutical compounding techniques. The carrier may take a wide variety of forms depending on the form of preparation desired for administration, e.g., oral or intravenous. In preparing the compositions for oral dosage form, any of the usual pharmaceutical media may be used, for example, water, glycols, oils, alcohols, flavouring agents, preservatives and colouring agents in the case of oral liquid preparations, for example, suspensions, elixirs and

solutions; or carriers such as starches, sugars, diluents, granulating agents, lubricants, binders and disintegrating agents in the case of oral solid preparations, for example, powders, capsules and tablets. Because of their ease of administration, tablets and capsules represent the most advantageous oral dosage unit form, in which case solid pharmaceutical carriers are obviously used. If desired, tablets may be sugar coated or enteric coated by standard techniques.

In addition to the ccmmon dosage forms set out above, the leukotriene inhibitors of Formula I may also be administered by controlled release means and/or delivery devices such as those described in Nos. US-A-3,845,770; 3,916,899; 3,536,809; 3,598,123; 3,630,200 and 4,008,719.

Dosage forms for application to treat the eye are also disclosed in US-A- 4,348,398.

Pharmaceutical compositions of the present invention suitable for oral administration and by inhalation in the case of asthma therapy may be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, lubricating, surface active or dispersing agent. Molded tablets may be made by molding in a suitable machine, a mixture of the powdered compound moistened with an inert liquid diluent. Desirably, each tablet contains from about 25 mg to about 500 mg of the active ingredient and each cachet or capsule contains from about 25 to about 500 mg of the active ingredient.

The following, prepared by conventional compounding procedures, are examples of representative pharmaceutical dosage forms:

| Injectible Suspension | mg/mL |
|---|---|
| Compound of Formula I | 1-100 |
| Methylcellulose | 5.0 |
| Tween 80 | 0.5 |
| Benzyl alcohol | 9.0 |
| Methyl paraben | 1.8 |
| Propyl paraben | 0.2 |
| Water for injection to a total volume of 1 ml | |

| Aerosol for Oral Inhibition | mg/can (200 doses/can) | |
|---|---|---|
| Compound of Formula I | 2-40 | |
| Oleic Acid | 0.2-4.0 | |
| Trichloromonofluoro methane | 5,000-8,000 | To a total of 20,400 |
| Dichloromonofluoro methane | 15,000-12,400 | |

| Cream | mg/g |
|---|---|
| Compound of Formula I | 1-100 |
| Cetyl alcohol | 130.0 |
| Sodium Lauryl Sulfate | 15.0 |
| Propylene Glycol | 100.0 |
| Methyl paraben | 1.8 |
| Propyl paraben | 1.2 |
| Purified Water of sufficient quantity to make total 1 g | |

| Ointment | mg/g |
|---|---|
| Compound of Formula I | 1-100 |
| Methyl paraben | 1.8 |
| Propyl paraben | 1.2 |
| Petrolatum of sufficient quantity to make total 1 g | |

| Tablet | mg/table |
|---|---|
| Compound of Formula I | 0.2-350 |
| Microcrystalline Cellulose | 0-349.8 |
| Providone | 14.0 |
| Microcrystalline Cellulose | 90.0 |
| Pregelatinized Starch | 43.5 |
| Magnesium Stearate | 2.5 |
| | 500 |

| Capsule | mg/capsule |
|---|---|
| Compound of Formula I | 0.2-350 |
| Lactose Powder | 248.5-598.3 |
| Magnesium Stearate | 1.5 |
| | 600 |

In addition to the compounds of Formula I, the pharmaceutical compositions can also contain other active ingredients, such as cyclooxygenase inhibitors, non-steroidal anti-inflammatory drugs (NSAIDs), peripheral analgesic agents such as zomepirac diflunisal and the like. The weight ratio of the compound of the Formula I to the second active ingredient may be varied and will depend upon the effective dose of each ingredient. Generally, an effective dose of each will be used. Thus, for example, when a compound of the Formula I is combined with an NSAID, the weight ratio of the compound of the Formula I to the NSAID will generally range from about 1000:1 to about 1:1000, preferably 200:1 to about 1:200. Combinations of a compound of the Formula I and other active ingredients will generally also be within the aforementioned range, but in each case, an effective dose of each active ingredient should be used.

Combinations of a compound of the Formula I and other active ingredients will generally be in the

aforementioned ratios.

NSAIDs can be characterized into five groups:

(1) the propionic acid derivatives;
(2) the acetic acid derivatives;
(3) the fenamic acid derivatives;
(4) the biphenylcarboxylic acid derivatives;
and
(5) the oxicams

or a pharmaceutically acceptable salt thereof.

The propionic acid derivatives which may be used comprise: ibuprofen, ibuprufen aluminum, indoprofen, ketoprofen, naproxen, benoxaprofen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen and bucloxic acid. Structurally related propionic acid derivatives having similar analgesic and anti-inflammatory properties are also intended to be included in this group.

Thus, "propionic acid derivatives" as defined herein are non-narcotic analgesics/non-steroidal anti-inflammatory drugs having a free $-CH(CH_3)COOH$ or $-CH_2CH_2COOH$ group (which optionally can be in the form of a pharmaceutically acceptable salt group, e.g., $-CH(CH_3)COO^-Na^+$ or $-CH_2CH_2COO^-Na^+$), typically attached directly or via a carbonyl function to a ring system, preferably to an aromatic ring system.

The acetic acid derivatives which may be used comprise: indomethacin, which is a preferred NSAID, sulindac, tolmetin, zomepirac, diclofenac, fenclofenac, alclofenac, ibufenac, isoxepac, furofenac, tiopinac, zidometacin, acemetacin, fentiazac, clidanac, oxpinac, and fenclozic acid. Structually related acetic acid derivatives having similar analgesic and antiinflammatory properties are also intended to be encompassed by this group.

Thus, "acetic acid derivatives" as defined herein are non-narcotic analgesics/non-steroidal anti-inflammatory drugs having a free $-CH_2COOH$ group (which optionally can be in the form of a pharmaceutically acceptable salt group, e.g. $-CH_2COO^-Na^+$), typically attached directly to a ring system, preferably to an aromatic or heteroaromatic ring system.

The fenamic acid derivatives which may be used comprise: mefenamic acid, meclofenamic acid, flufenamic acid, niflumic acid and tolfenamic acid. Structurally related fenamic acid derivatives having similar analgesic and anti-inflammatory properties are also intended to be encompassed by this group.

Thus, "fenamic acid derivatives" as defined herein are non-narcotic analgesics/non-steroidal anti-inflammatory drugs which contain the basic structure:

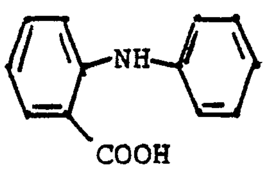

which can bear a variety of substituents and in which the free -COOH group can be in the form of a pharmaceutically acceptable salt group, e.g., $-COO^-Na^+$.

The biphenylcarboxylic acid derivatives which can be used comprise: diflunisal and flufenisal. Structurally related biphenylcarboxylic acid derivatives having similar analgesic and anti-inflammatory properties are also intended to be encompassed by this group.

Thus, "biphenylcarboxylic acid derivatives" as defined herein are non-narcotic analgesics/non-steroidal anti-inflammatory drugs which contain the basic structure:

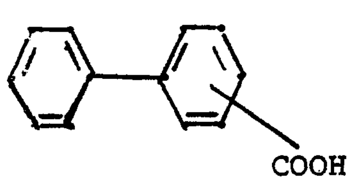

which can bear a variety of substituents and in which the free -COOH group can be in the form of a pharmaceutically acceptable salt group, e.g., $-COO^-Na^+$.

The oxicams which can be used in the present invention comprise: piroxicam, sudoxicam, isoxicam and 4-hydroxyl-1,2-benzothiazine 1,1-dioxide 4-(N-phenyl)-carboxamide. Structurally related oxicams having similar analgesic and anti-inflammatory properties are also intended to be encompassed by this group.

Thus, "oxicams" as defined herein are non-narcotic analgesics/non-steroidal anti-inflammatory drugs which have the general formula:

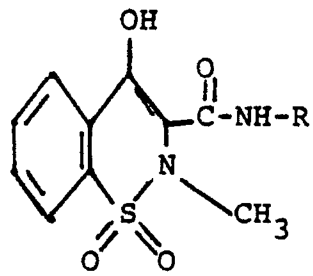

wherein R is an aryl or heteroaryl ring system.

The following NSAIDs may also be used: acemetacin, alminoprofen, amfenac sodium, aminoprofen, anitrazafen, antrafenine, auranofin, bendazac lysinate, benzydamine, beprozin, broperamole, bufezolac, carprofen, cinmetacin, ciproquazone, clidanac, cloximate, dazidamine, deboxamet, delmetacin, detomidine, dexindoprofen, diacerein, di-fisalamine, difenpyramide, emorfazone, enfenamic acid, enolicam, epirizole, etersalate, etodolac, etofenamate, fanetizole mesylate, fenclofenac, fenclorac, fendosal, fenflumizole, fentiazac, feprazone, floctafenine, flunixin, flunoxaprofen, fluproquazone, fopirtoline, fosfosal, furcloprofen, furofenac, glucametacin, guaimesal, ibuproxam, isofezolac, isonixim, isoprofen, isoxepac, isoxicam, lefetamine HCl, leflunomide, lofemizole, lonazolac calcium, lotifazole, loxoprofen, lysin clonixinate, meclofenamate sodium, meseclazone, miroprofen, nabumetone, nictindole, nimesulide, orpanoxin, oxametacin, oxapadol, oxaprozin, perisoxal citrate, pimeprofen, pimetacin, piproxen, pirazolac, pirfenidone, pirprofen, pranoprofen, proglumetacin maleate, proquazone, pyridoxiprofen, sudoxicam, suprofen, talmetacin, talniflumate, tenoxicam, thiazolinobutazone, thielavin B, tiaprofenic acid, tiaramide HCl, tiflamizole, timegadine, tioxaprofen, tolfenamic acid, tolpadol, tryptamid, ufenamate, and zidometacin.

The following NSAIDs, designated by company code number, may also be used:

480156S, AA861, AD1590, AFP802, AFP860, AI77B, AP504, AU8001, BPPC, BW540C, CHINOIN 127, CN100, EB382, EL508, F1044, GV3658, ITF182, KCNTEI6090, KME4, LA2851, MR714, MR897, MY309, ONO3144, PR823, PV102, PV108, R830, RS2131, SCR152, SH440, SIR133, SPAS510, SQ27239, ST281, SY6001, TA60, TVX2706, U60257, UR2301, and WY41770.

Finally, NSAIDS which may also be used include the salicylate, specifically aspirin, and the phenylbutazones, and pharmaceutically acceptable salts thereof.

Pharmaceutical compositions comprising the Formula I compounds may also contain inhibitor of the biosynthesis of the leukotrienes such as are disclosed in pending US Patent Applications Nos 539,342, filed October 5, 1983, 459,924, filed January 21, 1983, 539,215, filed October 5, 1983, and 547,161, filed October 31, 1983 which correspond respectively to European Patent Specifications EP-A-0 138 481, 0 115 394, 0 136 893 and 0 140 709.

The compounds of the Formula I may also be used in combination with leukotriene antagonists such as those disclosed in copendng US applications Nos 520,051 and 520,052, filed August 5, 1983 and others known in the art such as those disclosed in European Patent Applications Nos EP-A-0 056 172 and 0 061 800; and in UK Patent Specification No GB-A-2 058 785.

Pharmaceutical compositions comprising the Formula I may also contain as the second active ingredient, antihistaminic agents such as benadryl, dramamine, histadyl or phenergan. Alternatively, they may include prostaglandin antagonists such as those disclosed in European Patent Application No EP-A-0 011 067 or thromboxane antagonits such as those disclosed in US-A-4,237,160. They may also contain histidine decarboxyase inhibitors such as a $\alpha$-fluoromethylhistidine, described in US-A-4,325,961. The compounds of the Formula I may also be advantageously combined with an $H_1$ or $H_2$-receptor antagonist, such as for instance cimetidine, ranitidine, terfenadine, famotidine, aminothiadiazoles disclosed in EP-A-40696 and like compounds, such as those disclosed in U.S. Patent Nos. US-A-4,283,408; 4,362,736 and 4,394,508. The pharmaceutical compositions may also contain a $K^+/H^+$ ATPase inhibitor such as omeprazole, which is disclosed in US-A-4,255,431.

Another embodiment of the present invention are certain novel compounds encompassed by Formula I.

These compounds are shown in Table IV.

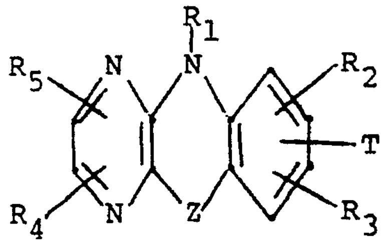

I

TABLE IV

NOVEL COMPOUNDS

| Z | $R_1$ | $R_2$ | $R_3$ | $R_4$ | $R_5$ | T |
|---|---|---|---|---|---|---|
| S | H | H | H | H | H | 7-$OCH_3$ |
| S | H | H | H | H | 8-Cl | 7-$OCH_3$ |
| S | $CH_3$ | H | H | H | 8-Cl | 7-$OCH_3$ |
| S | $CH_3$ | H | H | H | 8-Cl | 7-OH |
| S | $CH_3$ | H | H | H | H | 7-$OCH_3$ |
| S | H | H | H | H | H | 7-OH |
| S | $CH_3$ | H | H | H | H | 7-OH |
| SO | H | H | H | H | H | 7-$OCH_3$ |
| SO | $CH_3$ | H | H | H | H | 7-$OCH_3$ |
| S | $SO_2$Ph-p-Me | H | H | H | H | 7-$OCH_3$ |
| $SO_2$ | H | H | H | H | H | 7-$OCH_3$ |
| S | $COCH_3$ | H | H | H | H | 7-$OCH_3$ |
| S | $COCH_3$ | H | H | H | H | H |
| $SO_2$ | H | H | H | H | H | H |
| S | $CH_2CO_2C_2H_5$ | H | H | H | H | H |
| S | $CH_2CO_2H$ | H | H | H | H | H |
| S | $CO_2CH_3$ | H | H | H | H | H |
| S | $CH=CHCO_2CH_3$ | H | H | H | H | H |

The following examples are provided to aid in the interpretation of the claims appearing below. They are not intended as a limitation upon the scope of said claims. All temperatures are in degrees Celsius.

EXAMPLE 1

Preparation of 1,4-diazaphenoxazine

Step A : Sodium (1.15 g) was dissolved in iso-propanol (250 ml) with brief refluxing. The solution was cooled to room temperature and 2,3-dichloropyrazine (7.45 g) and ortho aminophenol (5.45 g) were added.

The mixture was heated at reflux for 4 hours, cooled to room temperature and evaporated to about one-quarter volume in vacuo . To the black solution was added water (200 ml) with stirring. A solid precipitate was collected by filtration. The brown solid was recrystallized from MeOH/$H_2O$ (2:1) to yield 6.3 g of fluffy brown needles. A second recrystallization afforded off-white needles, m.p. 88-89°.
Analysis, Calculated : C, 54.19; H, 3.64; N, 18.96; Cl, 15.99.
Observed : C, 54.25; H, 3.54; N, 18.60; Cl, 15.76.

Step B : A mixture of the compound from Step A (1 g) in pyridine (20 ml) was refluxed for 4 hours. DBU (2 ml) was added and the solution refluxed an additional 5 hours. The reaction mixture was diluted by the slow addition of water (100 ml). The resulting precipitate was collected by filtration, washed with water and air dried. Recrystallization from ethanol afforded the title compound, m.p. 230-232°.
Analysis, Calculated : C, 64.86; H, 3.81; N, 22.69.
Observed : C, 64.93; H, 3.80; N, 22.44.

## EXAMPLE 2

1,4-Diazaphenothiazine
Step A: Preparation of ortho-(2-chloro-3-pyrazinyl)-thioaniline

To a solution of 2,3-dichloropyrazine (7.45 g, 50 mmoles) and ortho-aminothiophenol (6.25 g, 50 mmoles) in tetrahydrofuran (50 ml) was added dropwise a solution of triethylamine (6 g) in THF (25 ml) over a period of 10 minutes. A cold water bath was used to control the modest exotherm and maintain the temperature below 20°. The triethylamine chloride was removed by filtration and the filtrate was evaporated to yield a solid that was slurried for 1 hour in methanol (75 ml). The solid thus obtained was recrystallized from methanol to yield the title compound, m.p. 130-135° (dec.).
Analysis, Calculated : C, 50.53; N, 3.39; N, 17.68; S, 13.49; Cl, 14.91
Observed : C, 50.68; N, 3.37; N, 17.56; S, 13.38; Cl, 14.54.

Step B: Preparation of 1,4-diazaphenothiazine

1,4-Diazaphenothiazine can be prepared by heating the title compound of Step A of this example with DBU in pyridine.

It was also prepared according to Cheeseman, Tetrahedron 33 827 (1973) to yield 1,4-diazaphenothiazine m.p. 190-193°. Lit. 190-192°.

## EXAMPLE 3

7-Methoxy-1,4-diaza-10H-phenothiazine

5-Methoxy-2-aminothiophenol (5.5 g, 35.4 mmole) 2,3-dichloropyrazine (5.4 g, 36.2 mmoles), sodium carbonate (7.8 g, 73.6 mmoles) in 30 ml o-dichlorobenzene was refluxed until a vigorous reaction took place. Heating was stopped, the reaction mixture was cooled to room temperature, poured in water (50 ml), stirred for 30 minutes, filtered, washed with water and air-dried to yield the title compound, m.p. 210-211° after recrystallization from ethanol-acetone.
Analysis, Calculated : C, 57.13; H, 3.92; N, 18.17; S, 13.86.
Observed : C, 57.23; H, 3.82; N, 17.94; S, 14.04.

## EXAMPLE 4

7-Methoxy-1,4-diaza-10-methylphenothiazine

10H-7-Methoxy-1,4-diazaphenothiazine (2.3 g, 10 mmoles) in glyme (50 ml) was heated with NaH (500

mg) when $N_2$ evolution subsided within 30 minutes, a red solution was obtained and treated with excess $CH_3I$ (5 ml). Reflux was maintained for 15 minutes. The mixture was absorbed on silica gel and elution with 30% EtOAc in hexane yield successively 1.8 g of the title compound, m.p. 109-111° and 0.23 g of the isomeric 7-methoxy-1-methyl-1,4-diazaphenothiazine, m.p. 128-130°.

Title compound:
Analysis, Calculated : C, 58.76; H, 4.52; N, 17.13; S, 13.07
Observed : C, 58.64; H, 4.48; N, 17.00; S, 12.92.
Isomeric compound:
Analysis, Calculated : C, 58,76; H, 4.52; N, 17.13; S, 13.07
Observed : C, 58.87; H, 4.68; N, 17.03; S, 13.15.

EXAMPLE 5

7-Hydroxy-1,4-diaza-10H-phenothiazine

Sodium hydride (60 mg, 2.5 mmoles) was added to ethanethiol (124 mg, 2 mmoles) in dry DMF (2 ml) under argon. 7-Methoxy-1,4-diaza-10H-phenothiazine (462 mg, 2 mmoles) was then added and the reaction mixture was refluxed for 3 hours. The mixture was poured into 0.5N HCl (50 ml). The solid was collected, washed with methanol and air-dried to yield starting material. Extraction of the filtrate with ethyl acetate gave 100 mg of a mixture that was chromatographed on silica gel, eluting with 40% ethyl acetate in hexane to yield 25 mg of the title compound.
Analysis, Calculated : C, 55.19; H, 3.25; N, 19.34; S, 14.76.
Observed : C, 55.19; H, 3.37; N, 19.12; S, 14.86.

EXAMPLE 6

7-Hydroxy-1,4-diaza-10-methylphenothiazine

Following a procedure identical to that described in Example 5, 7-methoxy-1,4-diaza-10-methylphenothiazine was converted to the title compound, m.p. 207-208.5°.
Analysis, Calculated : C, 57.13; H, 3.92; N, 18.17; S, 13.86
Observed : C, 57.16; H, 3.97; N, 18.26; S, 13.81.

EXAMPLE 7

7-Methoxy-1,4-diaza-10H-10-acetyl phenothiazine

A mixture of 7-methoxy-1,4-diaza-10H-phenothiazine (4.62 g, 20 mmoles) and sodium acetate (2.5 g) in acetic anhydride (50 ml) was refluxed for 18 hours. The reaction mixture was cooled to room temperature. The resulting crystals were filtered, washed with dilute acetic acid and air dried to yield 5.0 g of the title compound, m.p. 221-223°.

EXAMPLE 8

7-Methoxy-1,4-diaza-10-methylphenothiazine

To a suspension of sodium hydride (0.5 g, 20.8 mmoles) in dimethoxy ethane (50 ml) was added 7-methoxy-1,4-diaza-10H-phenothiazine (2.3 g, 10 mmoles). After stirring for 30 minutes, a deep red colored solution of the anion was obtained and was quenched by adding excess methyl iodide (5 ml). The reaction mixture was poured on ice and the resulting mixture was extracted with ethyl acetate, washed with brine,

dried ($Na_2SO_4$) and evaporated to dryness. The residue was chromatographed on silica gel, yielding 1.8 g of the title compound, m.p. 109-111°.

Also obtained was 0.28 g of isomeric 3-methoxy-6,9-diaza-9-methylphenothiazine, m.p. 128-130°.

EXAMPLE 9

1,4-Diaza-10-methylphenothiazine

Similarly, using the procedure of Example 8, substituting 1,4-diaza-10H-phenothiazine for 3-methoxy-1,4-diaza-10H-phenothiazine, the title compound, m.p. 101-102°, was obtained.

The isomeric 1,4-diaza-1-methylphenothiazine, m.p. 101-102° was also obtained.

EXAMPLE 10

1,4-Diazaphenothiazine-5-oxide

To 1,4-diaza-10H-phenothiazine (1 g, 5 mmoles) in acetic acid (15 ml) was added 50% hydrogen peroxide (1 ml). The mixture was stirred at 55° for 30 minutes. The mixture was evaporated to a small volume and the title compound crystallized out. The crystals were filtered, washed with acetic acid and air dried to yield 983 mg of the title compound, m.p. 261-263°.

EXAMPLE 11

7-Methoxy-1,4-diazaphenothiazine-5-oxide

Similarly, as in Example 10, but substituting 7-methoxy-1,4-diazaphenothiazine for 1,4-diazaphenothiazine, the title compound, m.p. 240-241°, was obtained in an 87% yield.

EXAMPLE 12

1,4-Diazaphenothiazine-5,5-dioxide

Similarly, by carrying out the reaction described in Example 10, but at 80° instead of 55°, the title compound, m.p. 316-318°, was obtained.

EXAMPLE 13

7-Methoxy-1,4-diazaphenothiazine-5,5-dioxide

Similarly, as in Example 10, but substituting 7-methoxy-1,4-diazaphenothiazine for 1,4-diazaphenothiazine and carrying out the reaction at 80° instead of 55°, the title compound, m.p. 293-294°, was obtained.

EXAMPLE 14

7-Methoxy-10-methyl-1,4-diazaphenothiazine-5-oxide

Similarly, as in Example 10, but substituting 7-methoxy-10-methyl-1,4-diazaphenothiazine for 1,4-diazaphenothiazine, the title compound, m.p. 229-231° was obtained in 67% yield.

EXAMPLE 15

1,4-Diaza-10-carboethoxymethylphenothiazine

Following the conditions of Example 4 but substituting ethyl bromoacetate for methyl iodide, the title compound, m.p. 115-117°, was obtained.

The isomeric 1,4-diaza-1-carboethoxymethyl phenothiazine m.p. 157-158°, was also obtained.

EXAMPLE 16

1,4-Diaza-10-carboxymethylphenothiazine

1,4-Diaza-10-carboethoxymethylphenothiazine (862 mg, 3 mmoles) in methanol (17 ml) was treated with 5N sodium hydroxide (1 ml). The mixture was refluxed for 2 hours. Water (17 ml) was added and the solution was acidified with 6N hydrochloric acid (1 ml). The title compound, m.p. 197-198°, was isolated by filtration in a yield of 84%.

EXAMPLE 17

10-Acetyl-1,4-diazaphenothiazine

By following the conditions of Example 7, but substituting 1,4-diazaphenothiazine for 3-methoxy-1,4-diazaphenothiazine, the title compound, m.p. 174-175°, was obtained in an 89% yield.

EXAMPLE 18

10-Carbomethoxy-1,4-diazaphenothiazine

By following the conditions of Example 4, but substituting 1,4-diazaphenothiazine for 7-methoxy 1,4-diazaphenothiazine and methylchloroformate for methyl iodide, the title compound, m.p. 137-138° was obtained in a 23% yield.

EXAMPLE 19

8-Chloro-1,4-diaza-7-methoxyphenothiazine

Following the conditions of Cheeseman et al., (Tetrahedron 33, 827 (1977)), the reaction of 2,3-dichloropyrazine with 2-amino-4-chloro-5-methoxythiophenol gave the title compound, in a 25% yield.
Analysis, Calculated : C, 4972; H, 3.03; N, 15.81; S, 13.34
Observed : C, 49.70; H, 2.83; N, 15.84; S, 13.54.

EXAMPLE 20

8-Chloro-7-methoxy-1,4-diaza-10-methylphenothiazine

By following the conditions of Example 4, but substituting 8-chloro-7-methoxy-1,4-diaza-phenothiazine for 7-methoxy-1,4-diazaphenothiazine, the title compound was obtained in a 50% yield.
Analysis, Calculated : C, 51.52; H, 3.60; N, 15.02; Cl, 12.67; S, 11.46
Observed : C, 51.66; H, 3.79; N, 14.84; Cl, 12.61; S, 11.71.

EXAMPLE 21

8-Chloro-7-hydroxy-1,4-diaza-10-methylphenothiazine

By following the conditions of Example 5, but substituting 8-chloro-7-methoxy-1,4-diaza-10-methyl phenothiazine for 7-methoxy-1,4-diazaphenothiazine, the title compound was obtained in a 90% yield.
Analysis, Calculated : C, 49.72; H, 3.03; N, 15.81; S, 12.07, Cl, 13.34
Observed : C, 49.60; H, 3.19; N, 15.75; S, 12.11; Cl, 13.21.

EXAMPLE 22

10-(2-Carbomethoxyvinyl)-1,4-diazaphenothiazine

To a suspension of sodium hydride (0.1 g, 4.2 mmoles) in dimethoxyethane (10 ml) was added 1,4-diazaphenothiazine (200 mg, 1 mmole). After stirring for 30 minutes a deep red solution of the anion was obtained and this was treated with methylpropiolate (85 µl, 1.0 mmole). Stirring was maintained for 30 minutes. The mixture was evaporated to dryness and the residue was purified by chromatography. Elution with 40% EtOAc/hexane gave the desired product as on orange solid, m.p. 165-169°. NMR analysis showed the product to be a mixture of trans and cis isomers in a ratio of 4 to 1.

EXAMPLE 23

7-Methoxy-10-p-toluenesulfonyl-1,4-diazaphenothiazine

To a cooled, stirred suspension of 1,4-diazaphenothiazine (2.31 g, 10 mmole) in glyme (23 ml) was added portionwise sodium hydride (300 mg). After stirring the mixture at room temperature for one hour, the mixture was again cooled in an ice-bath and p-tosylchloride (1.9 g, 10 mmole) was added in portions. An additional 200 mg of sodium hydride was added and the solution stirred at room temperature for one hour. Water was cautiously added, and the phases were separated. The organic phase was washed with water and dried. The title compound slowly crystallized from the organic solvent. The crude title compound was recrystallized from ethyl acetate/ diisopropyl ether (2:1).
Analysis, Calculated : C, 56.09; H, 3.92; H, 10.90; S, 16.64
Observed : C, 56.03; H, 4.00; N, 10.83; S, 16.81.

EXAMPLE 24

9-Oxo-10-trifluoroacetyl-9,10-dihydro-pyrazino[2,3-b] pyrrolo[1,2,3-d,e] [1,4]benzothiazine

A suspension of the acid from Example 16 (2.59 g, 10 mmole) in methylene chloride (78 ml) was treated with trifluoroacetic anhydride (1.55 ml, 2.31 g, 11.0 mmole). The mixture was stirred at room temperature overnight, and then evaporated. The residue was dissolved in ethylene chloride and the solution evaporated onto silica gel (13 g). The solid was placed on a column of silica gel (100 g) and elution with 1:1 ethyl acetate/hexane gave 878 mg of title compound.

Analysis, Calculated : C, 49.86; H, 1.79; N, 12.46; S, 9.50; F, 16.90
Observed : C, 49.69; H, 2.02; N, 12.22; S, 10.07; F: 16.67.

In those instances where asymmetric centers are present, more than one stereoisomer is possible, and all possible isomeric forms are deemed to be included within the planar structural representation shown. Optically active (R) and (S) isomers may be resolved using conventional techniques known to the skilled artisan.

## Claims

1. A pharmaceutical composition for inhibiting leukotriene biosynthesis or action comprising a compound of the Formula I:

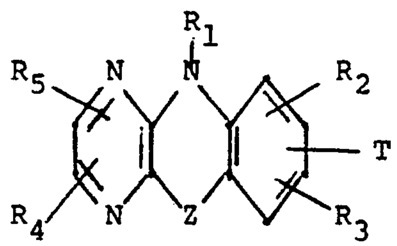

I

or a compound that is a pharmaceutically acceptable salt thereof, together with a pharmaceutically acceptable carrier, where, in the formula,

Z is O, NCN, S ,SO or $SO_2$;

$R_1$ is H, $C_{1-6}$ alkyl, benzyl, $C_{1-6}$ acyl, $C_{1-6}$ aminoacyl, ($C_{1-6}$ alkylacyloxy)-($C_{1-6}$ alkyl), ($C_{1-6}$ alkoxy)-($C_{1-6}$ alkyl), $(CH_2)_nCOOR_6$ where n is 0, 1, 2, 3 or 4, CN, ($C_{1-6}$ alkyl)acyloxy-($C_{1-6}$ alkoxy)-carbonyl, $-C(R_7)=C(R_7)COOR_6$ or $SO_2R_{10}$;

each of $R_2$, $R_3$, $R_4$ and $R_5$, independently of the others, is hydrogen, $C_{1-6}$ alkyl, $C_{2-6}$ alkenyl or $(CH_2)_nM$ where n is 0 or an integer from 1 to 6 and

M is

(a) $OR_{16}$
(b) halogen;
(c) $CF_3$;
(d) $SR_{16}$;
(e) phenyl;
(f) substituted phenyl where the substituents(s) is/are $C_{1-3}$ alkyl, halogen, CN, $C_{1-3}$ alkoxy, OH, $(CH_2)_nNR_8R_9$ where n is 0, 1 or 2, $CF_3$, $COOR_6$ or $CH_2COOR_6$; (herein called "substituted phenyl as defined");
(g) $COOR_6$;
(h) $-CO-R_{14}$;
(i) tetrazolyl;
(j) $NH-CO-R_7$;
(k) $NR_8R_9$;
(l) $NHSO_2R_{10}$;
(m) $-CO-CH_2OH$;
(n) $SOR_{11}$ where $R_{11}$ is $C_{1-6}$ alkyl, phenyl, substituted phenyl as defined, $(CH_2)_mCOOR_6$, where m is an integer from 1 to 6, or $CF_3$;
(o) $CONR_8R_9$;
(p) $SO_2NR_8R_9$;
(q) $SO_2R_{13}$ where $R_{13}$ is OH, $C_{1-6}$ alkyl, H, phenyl, substituted phenyl as defined, $(CH_2)_mCOOR_6$ or $CF_3$;
(r) $NO_2$;
(s) $-OCO-R_{14}$;
(t) $-OCO-NR_8R_9$;

(u) -OCO-$OR_7$; or

(v) -CN;

each $R_{16}$, independently of any other, is H; ($C_{1-6}$ alkoxy)-($C_{1-6}$ alkyl); ($C_{1-6}$ alkyl)acyloxy-($C_{1-6}$ alkyl); $C_{1-6}$ alkyl; substituted phenyl as defined; $-(CH_2)_mCOOR_6$; CN: $C_{1-5}$ alkylacyl; $C_{1-4}$ perfluoroalkyl; phenyl; benzyl; or $CH_2$-$R_{12}$ where $R_{12}$ is $C_{1-5}$ alkyldimethylamino;

each $R_6$, independently of any other, is H, $C_{1-6}$ alkyl, benzyl or phenyl;

each $R_{14}$, independently of any other, is H, $C_{1-6}$ alkyl, $C_{1-6}$ alkoxy, $C_{1-6}$ alkyl)acyloxy-($C_{1-6}$ alkoxy), $(CH_2)_nCOOR_6$ where n is 0 or an integer from 1 to 4, phenyl or substituted phenyl as defined or is such that $R_{14}COOH$ is an essential amino acid;

each of $R_8$ and $R_9$, independently of the others, is H, phenyl, substituted phenyl as defined or $C_{1-4}$ alkyl, or $NR_8R_9$ is a heterocycloalkyl of 5 to 8 ring atoms; and

each $R_7$, independently of the others, is H, $C_{1-6}$ alkyl, benzyl, phenyl or ($C_{1-6}$ alkyl)acyloxy-($C_{1-6}$ alkoxy);

each $R_{10}$ is independently OH, $C_{1-6}$ alkyl, $C_{1-6}$ alkoxy, phenyl or p-tolyl;

or any two of $R_1$, $R_2$, $R_3$, $R_4$ or $R_5$ are joined to form an additional ring of 5 to 7 members that optionally contains a carbonyl group and/or a hydroxyl group as substituent(s), and that has 0, 1 or 2 double bonds, and such that, if $R_1$ is a constituent of the ring, one member is nitrogen and the others are carbon and if $R_1$ is not a constituent of the ring, all the members are carbon; and

T is hydrogen or $OR_{15}$ where $R_{15}$ is hydrogen, $C_{1-6}$ alkyl, $C_{1-6}$ alkylacyl, phenyl-($C_{1-8}$ alkyl)acyl, $SO_2R_{10}$, arylsulfonyl, -CO-phenyl or substituted phenyl as defined.

**2.** A composition as claimed in Claim 1 in which Z is O.

**3.** A composition as claimed in Claim 1 in which Z is S,SO or $SO_2$.

**4.** A composition as claimed in Claim 1 in which Z is NCN.

**5.** A composition as claimed in Claim 1 in which Z is O, S,SO or $SO_2$, n is 0 in the unit $(CH_2)_nM$ and the remaining substituents are as defined in Claim 1.

**6.** A composition as claimed in Claim 1 in which Z is O, S,SO or $SO_2$, n is 1 in the unit $(CH_2)_nM$ and the remaining substituents are as defined in Claim 1.

**7.** A composition as claimed in Claim 1, in which

Z is O or S;

$R_1$ is H, $C_{1-6}$ alkyl, $C_{1-6}$ acyl, $-(CH_2)_nCOOR_6$, ($C_{1-6}$ alkyl)acyloxy -($C_{1-6}$ alkoxy)carbonyl, -C(H)=C-(H)$COOR_6$ or $SO_2R_{10}$;

each of $R_2$, $R_3$, $R_4$ and $R_5$, independently of the others, is hydrogen, $C_{1-6}$ alkyl, or $-(CH_2)_nM$ wherein: n is 0 or an integer from 1 to 6 and

M is

(a) $OR_{16}$

(b) halogen;

(c) $CF_3$;

(d) $NO_2$;

(e) -OCO-$R_{14}$;

(f) -OCO-$NR_8R_9$; or

(g) -OCO-$OR_7$; and the other variables are as defined in Claim 1, but with the proviso that there is a group $OR_{16}$ located at one of the 6,7,8 and 9-positions.

**8.** A composition as claimed in Claim 1, in which the variables in the formula are as follows:

| Z | $R_1$ | $R_2$ | $R_3$ | $R_4$ | $R_5$ | T |
|---|---|---|---|---|---|---|
| S | H | H | H | H | H | H |
| S | $CH_3$ | H | H | H | H | H |
| S | $CH_2Ph$ | H | H | H | H | H |
| S | H | $3\text{-}NO_2$ | H | H | H | H |
| S | H | 2-Cl | 3-Cl | H | H | H |
| S | H | $3\text{-}NO_2$ | $7\text{-}NO_2$ | H | H | H |
| S | $CH_3$ | $2\text{-}NO_2$ | H | H | H | H |
| S | $CH_3$ | $3\text{-}NO_2$ | H | H | H | H |
| S | $n\text{-}C_3H_7$ | 2-Cl | 3-Cl | H | H | H |
| S | $n\text{-}C_6H_{13}$ | 2-Cl | 3-Cl | H | H | H |
| S | $CH_2Ph$ | 2-Cl | 3-Cl | H | H | H |
| SO | H | H | H | H | H | H |
| SO | $CH_3$ | H | H | H | H | H |
| SO | H | 2-Cl | 3-Cl | H | H | H |
| SO | H | $3\text{-}NO_2$ | $7\text{-}NO_2$ | H | H | H |
| $SO_2$ | $CH_3$ | H | H | H | H | H |
| $SO_2$ | $CH_2Ph$ | H | H | H | H | H |
| $SO_2$ | $CH_2Ph$ | 2-Cl | 3-Cl | H | H | H |
| O | H | H | H | H | H | H |
| O | H | 2-F | 3-F | H | H | H |
| O | H | 2-Cl | 3-Cl | H | H | H |
| O | H | 2-Br | 3-Br | H | H | H |
| O | H | 2-Cl | 3-Cl | $8\text{-}CH_3$ | H | H |
| N-CN | H | H | H | H | H | H |
| N-CN | $CH_3$ | $2\text{-}CH_3$ | $3\text{-}CH_3$ | H | H | H |
| N-CN | $CH_3$ | H | H | H | H | $7\text{-}OCH_3$ |
| S | H | H | H | H | H | $7\text{-}OCH_3$ |
| S | H | H | H | H | 8-Cl | $7\text{-}OCH_3$ |

| Z | $R_1$ | $R_2$ | $R_3$ | $R_4$ | $R_5$ | T |
|---|---|---|---|---|---|---|
| S | $CH_3$ | H | H | H | 8-Cl | 7-$OCH_3$ |
| S | $CH_3$ | H | H | H | 8-Cl | 7-OH |
| S | $CH_3$ | H | H | H | H | 7-$OCH_3$ |
| S | H | H | H | H | H | 7-OH |
| S | $CH_3$ | H | H | H | H | 7-OH |
| SO | H | H | H | H | H | 7-$OCH_3$ |
| SO | $CH_3$ | H | H | H | H | 7-$OCH_3$ |
| S | $SO_2$Ph-p-Me | H | H | H | H | 7-$OCH_3$ |
| $SO_2$ | H | H | H | H | H | 7-$OCH_3$ |
| S | $COCH_3$ | H | H | H | H | 7-$OCH_3$ |
| S | $COCH_3$ | H | H | H | H | H |
| $SO_2$ | H | H | H | H | H | H |
| S | $CH_2CO_2C_2H_5$ | H | H | H | H | H |
| S | $CH_2CO_2H$ | H | H | H | H | H |
| S | $CO_2CH_3$ | H | H | H | H | H |
| S | $CH=CHCO_2CH_3$ | H | H | H | H | H |
| S | H | 2-Cl | H | H | H | H |
| S | H | 3-Cl | H | H | H | H |
| S | H | 8-Cl | H | H | H | H |
| S | H | 2-Me | H | H | H | H |

9. A composition as claimed in Claim 1 additionally comprising a second active ingredient that is a non-steroidal anti-inflammatory drug; a peripheral analgesic agent; a cyclooxygenase inhibitor; a leukotriene antagonist; an antihistaminic agent; a prostaglandin antagonist or a thromboxane antagonist.

10. A composition as claimed in Claim 9 in which the second active ingredient is a non-steroidal anti-inflammatory drug.

11. A composition as claimed in Claim 10 in which the non-steroidal anti-inflammatory drug is indomethacin.

12. A composition as claimed in Claim 9 in which the weight ratio of the compound of Claim 1 to the second active ingredient ranges from 1000:1 to 1:1000.

13. A composition as claimed in Claim 12 in which the ratio ranges from 200:1 to 1:200.

14. A composition as claimed in any one of Claims 1 to 8 or a compound having the formula set forth in Claim 1 with the variables as defined in any one of Claims 1 to 8, for use in the therapeutic treatment of mammals, especially humans.

15. A composition as claimed in any one of Claims 1 to 8 or a compound having the formula set forth in Claim 1 with the variables as defined in any one of Claims 1 to 8, for use in (a) inhibiting mammalian leukotriene biosynthesis or action; (b) treating or ameliorating pulmonary conditions; (c) treating inflammation; (d) treating allergies; or (e) treating skin conditions.

**16.** Compounds having the Formula I:

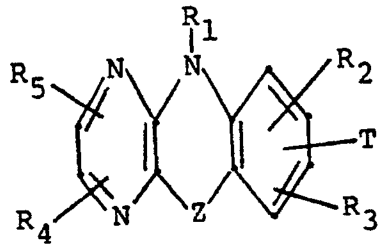

I

in which the variables are

| Z | $R_1$ | $R_2$ | $R_3$ | $R_4$ | $R_5$ | T |
|---|---|---|---|---|---|---|
| S | H | H | H | H | H | 7-$OCH_3$ |
| S | H | H | H | H | 8-Cl | 7-$OCH_3$ |
| S | $CH_3$ | H | H | H | 8-Cl | 7-$OCH_3$ |
| S | $CH_3$ | H | H | H | 8-Cl | 7-OH |
| S | $CH_3$ | H | H | H | H | 7-$OCH_3$ |
| S | H | H | H | H | H | 7-OH |
| S | $CH_3$ | H | H | H | H | 7-OH |
| SO | H | H | H | H | H | 7-$OCH_3$ |
| SO | $CH_3$ | H | H | H | H | 7-$OCH_3$ |
| S | $SO_2$Ph-p-Me | H | H | H | H | 7-$OCH_3$ |
| $SO_2$ | H | H | H | H | H | 7-$OCH_3$ |
| S | $COCH_3$ | H | H | H | H | 7-$OCH_3$ |
| S | $COCH_3$ | H | H | H | H | H |
| $SO_2$ | H | H | H | H | H | H |
| S | $CH_2CO_2C_2H_5$ | H | H | H | H | H |
| S | $CH_2CO_2H$ | H | H | H | H | H |
| S | $CO_2CH_3$ | H | H | H | H | H |
| S | $CH=CHCO_2CH_3$ | H | H | H | H | H |

**17.** 7-Methoxy-1,4-diaza-10-methylphenothiazine.

**18.** 7-Hydroxy-1,4-diaza-10H-phenothiazine.

**19.** 7-Hydroxy-1,4-diaza-10-methylphenothiazine.

**20.** 7-Methoxy-1,4-diaza-10-methylphenothiazine.

**21.** 8-Chloro-1,4-diaza-7-methoxyphenothiazine.

**22.** 8-Chloro-7-methoxy-1,4-diaza-10-methylphenothiazine.

**23.** 8-Chloro-7-hydroxy-1,4-diaza-10-methylphenothiazine.

## Revendications

**1.** Composition pharmaceutique pour inhiber l'action ou la biosynthèse des leucotriènes, qui comprend un composé de formule I :

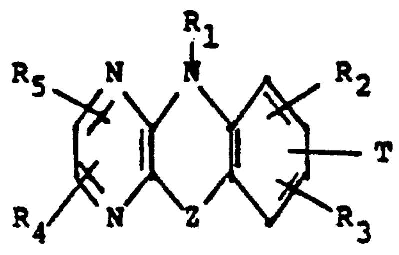

I

ou un composé qui est un de ses sels pharmaceutiquement acceptables, conjointement avec un véhicule pharmaceutiquement acceptable, où dans la formule,

Z est O, NCN, S, SO ou $SO_2$ ;

$R_1$ est H, un groupe alkyle en $C_{1-6}$, benzyle, acyle en $C_{1-6}$, aminoacyle en $C_{1-6}$, (alkylacyloxy en $C_{1-6}$)-(alkyle en $c_{1-6}$), (alcoxy en $C_{1-6}$)-(alkyle en $C_{1-6}$), $-(CH_2)_nCOOR_6$ où n vaut 0, 1, 2, 3 ou 4, CN, (alkyle en $C_{1-6}$)acyloxy-(alcoxy en $C_{1-6}$)carbonyle, $-C(R_7)=C(R_7)COOR_6$ ou $SO_2R_{10}$ ;

$R_2$, $R_3$, $R_4$ et $R_5$ représentent chacun, indépendamment des autres, un atome d'hydrogène, un groupe alkyle en $C_{1-6}$, alcényle en $C_{2-6}$ ou $-(CH_2)_nM$ ou n vaut 0 ou un nombre entier allant de 1 à 6, et

M est

- (a) $OR_{16}$
- (b) un atome d'halogène ;
- (c) $CF_3$ ;
- (d) $SR_{16}$ ;
- (e) le groupe phényle
- (f) un groupe phényle substitué dans lequel le(s) substituant(s) est/sont un groupe alkyle en $C_{1-3}$, un atome d'halogène, un groupe CN, alcoxy en $C_{1-3}$, OH, $(CH_2)_nNR_8R_9$ où n vaut 0, 1 ou 2, $CF_3$, $COOR_6$ ou $CH_2COOR_6$ ; (appelé ici le "groupe phényle susbtitué tel que défini")
- (g) $COOR_6$ ;
- (h) $-CO-R_{14}$
- (i) le groupe tétrazolyle ;
- (j) $NH-CO-R_7$ ;
- (k) $NR_8R_9$
- (l) $NHSO_2R_{10}$
- (m) $-CO-CH_2OH$ ;
- (n) $SOR_{11}$ où $R_{11}$ est un groupe alkyle en $C_{1-6}$, phényle, phényle substitué tel que défini, $(CH_2)_mCOOR_6$, où m est un nombre entier allant de 1 à 6, ou $CF_3$ ;
- (o) $CONR_8R_9$ ;
- (P) $SO_2NR_8R_9$ ;
- (q) $SO_2R_{13}$ où $R_{13}$ est OH, un groupe alkyle en $C_{1-6}$, H, le groupe phényle, phényle substitué tel que défini, $(CH_2)_mCOOR_6$ ou $CF_3$ ;
- (r) $NO_2$ ;
- (s) $-OCO-R_{14}$ ;
- (t) $-OCO-NR_8R$ ;
- (u) $-OCO-OR_7$ ; ou
- (v) -CN ;

chaque $R_{16}$, indépendamment des autres est H; un groupe (alcoxy en $C_{1-6}$)-(alkyle en $c_{1-6}$) ; (alkyle en $C_{1-6}$)acyloxy-(alkyle en $C_{1-6}$) ; alkyle en $C_{1-6}$ ; phényle substitué tel que défini ; $-(CH_2)_mCOOR_6$

; CN ; alkylacyle en $C_{1-5}$, perfluoroalkyle en $C_{1-4}$ ; phényle, benzyle ou $CH_2$-$R_{12}$ où $R_{12}$ est un groupe alkyldiméthylamino en $C_{1-5}$ ;
chaque $R_6$, indépendamment des autres est H, un groupe alkyle en $C_{1-6}$, benzyle ou phényle ;
chaque $R_{14}$, indépendamment des autres est H, un groupe alkyle en $C_{1-6}$, alcoxy en $C_{1-6}$, (alkyle en $C_{1-6}$)acyloxy-(alcoxy en $C_{1-6}$), $(CH_2)_nCOOR_6$ où n vaut 0 ou un nombre entier de 1 à 4, phényle ou phényle substitué tel que défini ou est tel que $R_{14}COOH$ est un acide aminé essentiel ;
$R_8$ et $R_9$ sont chacun, indépendamment les uns des autres H, un groupe phényle, phényle substitué tel que défini ou alkyle en $C_{1-4}$ ou $NR_8R_9$ est un groupe hétérocycloalkyle avec 5 à 8 atomes cycliques ; et
chaque $R_7$ , indépendamment des autres, est H, un groupe alkyle en $C_{1-6}$, benzyle, phényle ou (alkyle en $C_{1-6}$)acyloxy-(alcoxy en $C_{1-6}$) ;
chaque $R_{10}$ est indépendamment OH, un groupe alkyle en $C_{1-6}$, alcoxy en $C_{1-6}$, phényle ou p-tolyle ;
ou deux quelconques d'entre $R_1$, $R_2$, $R_3$ $R_4$ ou $R_5$ sont reliés pour former un cycle supplémentaire de cinq à sept chaînons qui contient éventuellement un groupe carbonyle et/ou un groupe hydroxyle à titre de substituant(s), et qui possède 0, 1 ou 2 double liaisons, et qui est tel que, si $R_1$ est un constituant du cycle, un représentant est l'azote et les autres sont des carbones et si $R_1$ n'est pas un constituant du cycle, tous les représentants sont des carbones ; et
T est un atome d'hydrogène ou un groupe $OR_{15}$ où $R_{15}$ est un atome d'hydrogène, un groupe alkyle en $C_{1-6}$, alkylacyle en $C_{1-6}$, phényl-(alkyle en $C_{1-8}$)acyle, $SO_2R_{10}$, arylsulfonyle, -CO-phényle ou phényle substitué tel que défini.

2. Composition selon le revendication 1, dans laquelle Z est O.

3. Composition selon la revendication 1, dans laquelle Z est S, SO ou $SO_2$.

4. Composition selon la revendication 1, dans laquelle Z est NCN.

5. Composition selon la revendication 1, dans laquelle Z est O, S, SO ou $SO_2$ n vaut 0 dans le motif --$(CH_2)_nM$ et les substituants restants sont tels que définis dans la revendication 1.

6. Composition selon la revendication 1, dans laquelle Z est O, S, SO ou $SO_2$, n vaut 1 dans le motif --$(CH_2)_nM$ et les susbtituants restants sont tels que définis dans la revendication 1.

7. Composition selon la revendication 1, dans laquelle
Z est O ou S ;
$R_1$ est H, un groupe alkyle en $C_{1-6}$, acyle en $C_{1-6}$, $-(CH_2)_nCOOR_6$, (alkyle en $C_{1-6}$)acyloxy-(alcoxy en $C_{1-6}$)carbonyle, $-C(H)=C(H)COOR_6$ ou $SO_2R_{10}$ ;
$R_2$, $R_3$, $R_4$ et $R_5$ sont chacun, indépendamment les uns des autres, un atome d'hydrogène, un groupe alkyle en $C_{1-6}$, ou $-(CH_2)_nM$, où :
n vaut 0 ou un nombre entier allant de 1 à 6 et
M est
(a) $OR_{16}$ ;
(b) un atome d'halogène ;
(c) $CF_3$ ;
(d) $NO_2$ ;
(e) -OCO-$R_{14}$ ;
(f) -OCO-$NR_8R_9$ ; ou
(g) -OCO-$R_7$ ; et les autres variables sont telles que définies dans la revendication 1, mais à la condition qu'il y ait un groupe $OR_{16}$ situé sur l'une des positions 6, 7, 8 et 9.

8. Composition selon la revendication 1, dans laquelle les variables de la formule sont les suivantes :

| Z | $R_1$ | $R_2$ | $R_3$ | $R_4$ | $R_5$ | T |
|---|---|---|---|---|---|---|
| S | H | H | H | H | H | H |
| S | $CH_3$ | H | H | H | H | H |
| S | $CH_2Ph$ | H | H | H | H | H |
| S | H | 3-$NO_2$ | H | H | H | H |
| S | H | 2-Cl | 3-Cl | H | H | H |
| S | H | 3-$NO_2$ | 7-$NO_2$ | H | H | H |
| S | $CH_3$ | 2-$NO_2$ | H | H | H | H |
| S | $CH_3$ | 3-$NO_2$ | H | H | H | H |
| S | n-$C_3H_7$ | 2-Cl | 3-Cl | H | H | H |
| S | n-$C_6H_{13}$ | 2-Cl | 3-Cl | H | H | H |
| S | $CH_2Ph$ | 2-Cl | 3-Cl | H | H | H |
| SO | H | H | H | H | H | H |
| SO | $CH_3$ | H | H | H | H | H |
| SO | H | 2-Cl | 3-Cl | H | H | H |
| SO | H | 3-$NO_2$ | 7-$NO_2$ | H | H | H |
| $SO_2$ | $CH_3$ | H | H | H | H | H |
| $SO_2$ | $CH_2Ph$ | H | H | H | H | H |
| $SO_2$ | $CH_2Ph$ | 2-Cl | 3-Cl | H | H | H |
| O | H | H | H | H | H | H |
| O | H | 2-F | 3-F | H | H | H |
| O | H | 2-Cl | 3-Cl | H | H | H |
| O | H | 2-Br | 3-Br | H | H | H |
| O | H | 2-Cl | 3-Cl | 8-$CH_3$ | H | H |
| N-CN | H | H | H | H | H | H |
| N-CN | $CH_3$ | 2-$CH_3$ | 3-$CH_3$ | H | H | H |
| N-CN | $CH_3$ | H | H | H | H | 7-$OCH_3$ |
| S | H | H | H | H | H | 7-$OCH_3$ |
| S | H | H | H | H | 8-Cl | 7-$OCH_3$ |

| Z | $R_1$ | $R_2$ | $R_3$ | $R_4$ | $R_5$ | T |
|---|---|---|---|---|---|---|
| S | $CH_3$ | H | H | H | 8-Cl | 7-$OCH_3$ |
| S | $CH_3$ | H | H | H | 8-Cl | 7-OH |
| S | $CH_3$ | H | H | H | H | 7-$OCH_3$ |
| S | H | H | H | H | H | 7-OH |
| S | $CH_3$ | H | H | H | H | 7-OH |
| SO | H | H | H | H | H | 7-$OCH_3$ |
| SO | $CH_3$ | H | H | H | H | 7-$OCH_3$ |
| S | $SO_2$Ph-p-Me | H | H | H | H | 7-$OCH_3$ |
| $SO_2$ | H | H | H | H | H | 7-$OCH_3$ |
| S | $COCH_3$ | H | H | H | H | 7-$OCH_3$ |
| S | $COCH_3$ | H | H | H | H | H |
| $SO_2$ | H | H | H | H | H | H |
| S | $CH_2CO_2C_2H_5$ | H | H | H | H | H |
| S | $CH_2CO_2H$ | H | H | H | H | H |
| S | $CO_2CH_3$ | H | H | H | H | H |
| S | $CH=CHCO_2CH_3$ | H | H | H | H | H |
| S | H | 2-Cl | H | H | H | H |
| S | H | 3-Cl | H | H | H | H |
| S | H | 8-Cl | H | H | H | H |
| S | H | 2-Me | H | H | H | H |

**9.** Composition selon la revendication 1, qui comprend en outre un second principe actif qui est un médicament anti-inflammatoire non stéroïdien ; un agent analgésique périphérique ; un inhibiteur de la cyclo-oxygènase ; un antagoniste des leucotriènes ; un agent antihistaminique ; un antagoniste des prostaglandines ou un antagoniste de la thromboxane.

**10.** Composition selon la revendication 9, dans laquelle le second principe actif est un médicament anti-inflammatoire non stéroïdien.

**11.** Composition selon la revendication 10, dans laquelle le médicament anti-inflammatoire non stéroïdien est l'indométacine.

**12.** Composition selon la revendication 9, dans laquelle le rapport pondéral du composé de la revendication 1 au second principe actif est compris dans l'intervalle de 1000 : 1 à 1 : 1000.

**13.** Composition selon la revendication 12, dans laquelle le rapport est compris entre 200 : 1 et 1 : 200.

**14.** Composition selon l'une quelconque des revendications 1 à 8, ou composé ayant la formule indiquée dans la revendication 1 avec les variables telles que définies dans l'une quelconque des revendications 1 à 8, à utiliser dans le traitement thérapeutique des mammifères, en particulier de l'homme.

**15.** Composition selon l'une quelconque des revendications 1 à 8 ou composé répondant à la formule indiquée dans la revendication 1 avec les variables telles que définies dans l'une quelconque des revendications 1 à 8, à utiliser (a) dans l'inhibition de l'action ou de la biosynthèse des leucotriènes chez

le mammifère ; (b) dans le traitement ou l'amélioration des états pulmonaires ; (c) dans le traitement de l'inflammation ; (d) dans le traitement des allergies ; ou (e) dans le traitement des états dermiques.

**16.** Composés répondant à la formule I :

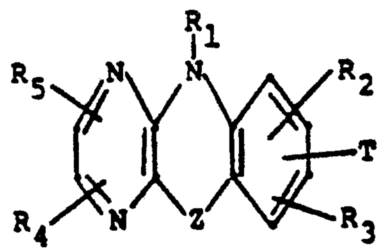

I

dans laquelle les variables sont :

| Z | $R_1$ | $R_2$ | $R_3$ | $R_4$ | $R_5$ | T |
|---|---|---|---|---|---|---|
| S | H | H | H | H | H | 7-$OCH_3$ |
| S | H | H | H | H | 8-Cl | 7-$OCH_3$ |
| S | $CH_3$ | H | H | H | 8-Cl | 7-$OCH_3$ |
| S | $CH_3$ | H | H | H | 8-Cl | 7-OH |
| S | $CH_3$ | H | H | H | H | 7-$OCH_3$ |
| S | H | H | H | H | H | 7-OH |
| S | $CH_3$ | H | H | H | H | 7-OH |
| SO | H | H | H | H | H | 7-$OCH_3$ |
| SO | $CH_3$ | H | H | H | H | 7-$OCH_3$ |
| S | $SO_2Ph$-p-Me | H | H | H | H | 7-$OCH_3$ |
| $SO_2$ | H | H | H | H | H | 7-$OCH_3$ |
| S | $COCH_3$ | H | H | H | H | 7-$OCH_3$ |
| S | $COCH_3$ | H | H | H | H | H |
| $SO_2$ | H | H | H | H | H | H |
| S | $CH_2CO_2C_2H_5$ | H | H | H | H | H |
| S | $CH_2CO_2H$ | H | H | H | H | H |
| S | $CO_2CH_3$ | H | H | H | H | H |
| S | $CH{=}CHCO_2CH_3$ | H | H | H | H | H |

**17.** 7-Méthoxy-1,4-diaza-10-méthylphénothiazine.

**18.** 7-Hydroxy-1,4-diaza-10H-phénothiazine

**19.** 7-Hydroxy-1,4-diaza-10-méthylphénothiazine.

**20.** 7-Méthoxy-1,4-diaza-10-méthylphénothiazine.

**21.** 8-Chloro-1,4-diaza-7-méthoxyphénothiazine.

**22.** 8-Chloro-7-méthoxy-1,4-diaza-10-méthylphénothiazine.

**23.** 8-Chloro-7-hydroxy-1,4-diaza-10-méthylphénothiazine.

## Ansprüche

**1.** Pharmazeutische Zusammensetzung zur Inhibierung der Leukotrienbiosynthese oder -wirkung, welche eine Verbindung der Formel I umfaßt:

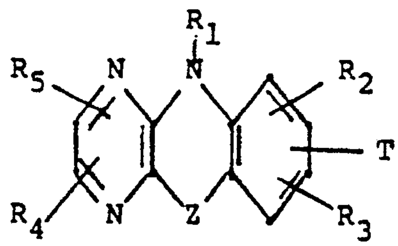

I

oder eine Verbindung, die ein pharmazeutisch annehmbares Salz davon ist, zusammen mit einem pharmazeutisch annehmbaren Träger, worin in der Formel

Z O, NCN, S, SO oder $SO_2$ ist;

$R_1$ H, $C_{1-6}$-Alkyl, Benzyl, $C_{1-6}$-Acyl, $C_{1-6}$-Aminoacyl, ($C_{1-6}$-Alkylacyloxy)($C_{1-6}$-alkyl), ($C_{1-6}$-Alkoxy) ($C_{1-6}$-Alkyl), -($CH_2$)n$COOR_6$, worin n 0, 1, 2, 3 oder 4 ist, CN, ($C_{1-6}$-Alkyl)acyloxy($C_{1-6}$-alkoxy)carbonyl, -C($R_7$) = C($R_7$)$COOR_6$ oder $SO_2R_{10}$ ist;

jedes von $R_2$, $R_3$, $R_4$ und $R_5$ unabhängig von den anderen Wasserstoff, $C_{1-6}$-Alkyl, $C_{2-6}$-Alkenyl oder -$(CH_2)_n$M ist, worin n 0 oder eine ganze Zahl von 1 bis 6 ist, und

M

- (a) $OR_{16}$
- (b) Halogen;
- (c) $CF_3$;
- (d) $SR_{16}$;
- (e) Phenyl;
- (f) substituiertes Phenyl, worin der/die Substituent/en $C_{1-3}$-Alkyl, Halogen, CN, $C_{1-3}$-Alkoxy, OH, $(CH_2)_nNR_8R_9$, worin n 0, 1 oder 2 ist, $CF_3$, $COOR_6$ oder $CH_2COOR_6$ ist/sind; (hier "substituiertes Phenyl, wie definiert" genannt);
- (g) $COOR_6$;
- (h) -CO-$R_{14}$;
- (i) Tetrazolyl;
- (j) NH-CO-$R_7$;
- (k) $NR_8R_9$;
- (l) $NHSO_2R_{10}$;
- (m) -CO-$CH_2$OH;
- (n) $SOR_{11}$, worin $R_{11}$ $C_{1-6}$-Alkyl, Phenyl, substituiertes Phenyl, wie definiert, $(CH_2)_mCOOR_6$, worin m eine ganze Zahl von 1 bis 6 ist, oder $CF_3$ ist;
- (o) $CONR_8R_9$;
- (p) $SO_2NR_8R_9$;
- (q) $SO_2R_{13}$, worin $R_{13}$ OH, $C_{1-6}$-Alkyl, H, Phenyl, substituiertes Phenyl, wie definiert, $(CH_2)_mCOOR_6$ oder $CF_3$ ist;
- (r) $NO_2$;
- (s) -OCO-$R_{14}$;
- (t) -OCO-$NR_8R_9$;
- (u) -OCO-$OR_7$; oder
- (v) -CN ist;

jedes $R_{16}$, unabhängig von jedem anderen, H; ($C_{1-6}$-Alkoxy) ($C_{1-6}$-Alkyl); ($C_{1-6}$-Alkyl)acyloxy($C_{1-6}$-alkyl); $C_{1-6}$-Alkyl; substituiertes Phenyl, wie definiert; $-(CH_2)_mCOOR_6$; CM; $C_{1-5}$-Alkylacyl; $C_{1-4}$-Per fluoralkyl; Phenyl; Benzyl; oder $CH_2$-$R_{12}$ ist, worin $R_{12}$ $C_{1-5}$-Alkyldimethylamino ist;

jedes $R_6$, unabhängig von jedem anderen, H, $C_{1-6}$-Alkyl, Benzyl oder Phenyl ist;
jedes $R_{14}$, unabhängig von jedem anderen, H, $C_{1-6}$-Alkyl, $C_{1-6}$-Alkoxy, ($C_{1-6}$-Alkyl)acyloxy($C_{1-6}$-alkoxy), $(CH_2)_nCOOR_6$, worin n 0 oder eine ganze Zahl von 1 bis 4 ist, Phenyl oder substituiertes Phenyl, wie definiert, ist oder so ist, daß $R_{14}COOH$ eine essentielle Aminosäure ist;
jedes von $R_8$ und $R_9$, unabhängig von den anderen, H, Phenyl, substituiertes Phenyl, wie definiert, oder $C_{1-4}$-Alkyl ist, oder $NR_8R_9$ ein Heterocycloalkyl mit 5 bis 8 Ringatomen ist; und
jedes $R_7$, unabhängig von den anderen, H, $C_{1-6}$-Alkyl, Benzyl, Phenyl oder ($C_{1-6}$-Alkyl)acyloxy ($C_{1-6}$-alkoxy) ist;
jedes $R_{10}$ unabhängig OH, $C_{1-6}$-Alkyl, $C_{1-6}$-Alkoxy, Phenyl oder p-Tolyl ist;
oder zwei beliebige von $R_1$, $R_2$, $R_3$, $R_4$ oder $R_5$ unter Bildung eines zusätzlichen Rings mit 5 bis 7 Gliedern zusammengenommen werden, welcher gegebenenfalls eine Carbonylgruppe und/oder eine Hydroxylgruppe als Substituent(en) enthält und der 0,1 oder 2 Doppelbindungen hat, so daß, wenn $R_1$ ein Bestandteil des Rings ist, ein Glied Stickstoff ist und die anderen Kohlenstoff sind, und daß, wenn $R_1$ kein Bestandteil des Rings ist, alle Glieder Kohlenstoff sind; und
T Wasserstoff oder $OR_{15}$ ist, worin $R_{15}$ Wasserstoff, $C_{1-6}$-Alkyl, $C_{1-6}$-Alkylacyl, Phenyl($C_{1-8}$-alkyl)-acyl, $SO_2R_{10}$, Arylsulfonyl, -CO-phenyl oder substituiertes Phenyl, wie definiert, ist.

**2.** Zusammensetzung nach Anspruch 1, worin Z O ist.

**3.** Zusammensetzung nach Anspruch 1, worin Z S, SO oder $SO_2$ ist.

**4.** Zusammensetzung nach Anspruch 1, worin Z NCN ist.

**5.** Zusammensetzung nach Anspruch 1, worin Z O, S, SO oder $SO_2$ ist, n in der Einheit $-(CH_2)_nM$ O ist und die verbleibenden Substituenten wie in Anspruch 1 definiert sind.

**6.** Zusammensetzung nach Anspruch 1, worin Z O, S, SO oder $SO_2$ ist, n in der Einheit $-(CH_2)_nM$ 1 ist und die verbleibenden Substituenten wie in Anspruch 1 definiert sind.

**7.** Zusammensetzung nach Anspruch 1, worin
Z O oder S ist;
$R_1$ H, $C_{1-6}$-Alkyl, $C_{1-6}$-Acyl, $-(CH_2)_nCOOR_6$, ($C_{1-6}$-Alkyl)acyloxy($C_{1-6}$-alkoxy)carbonyl, -C(H) = C(H)-$COOR_6$ oder $SO_2R_{10}$ ist;
jedes von $R_2$, $R_3$, $R_4$ und $R_5$ unabhängig von den anderen Wasserstoff, $C_{1-6}$-Alkyl oder $-(CH_2)_nM$ ist, worin
n 0 oder eine ganze Zahl von 1 bis 6 ist und
M
- (a) $OR_{16}$;
- (b) Halogen;
- (c) $CF_3$;
- (d) $NO_2$;
- (e) -OCO-$R_{14}$;
- (f) -OCO-$NR_8R_9$; oder
- (g) -OCO-$OR_7$ ist; und die anderen Variablen wie in Anspruch 1 definiert sind, jedoch mit der Maßgabe, daß eine Gruppe $OR_{16}$ an einer der Positionen 6, 7, 8 und 9 lokalisiert ist.

**8.** Zusammensetzung nach Anspruch 1, worin die Variablen in der Formel wie folgt sind:

| Z | $R_1$ | $R_2$ | $R_3$ | $R_4$ | $R_5$ | T |
|---|---|---|---|---|---|---|
| S | H | H | H | H | H | H |
| S | $CH_3$ | H | H | H | H | H |
| S | $CH_2Ph$ | H | H | H | H | H |
| S | H | 3-$NO_2$ | H | H | H | H |
| S | H | 2-Cl | 3-Cl | H | H | H |
| S | H | 3-$NO_2$ | 7-$NO_2$ | H | H | H |
| S | $CH_3$ | 2-$NO_2$ | H | H | H | H |
| S | $CH_3$ | 3-$NO_2$ | H | H | H | H |
| S | n-$C_3H_7$ | 2-Cl | 3-Cl | H | H | H |
| S | n-$C_6H_{13}$ | 2-Cl | 3-Cl | H | H | H |
| S | $CH_2Ph$ | 2-Cl | 3-Cl | H | H | H |
| SO | H | H | H | H | H | H |
| SO | $CH_3$ | H | H | H | H | H |
| SO | H | 2-Cl | 3-Cl | H | H | H |
| SO | H | 3-$NO_2$ | 7-$NO_2$ | H | H | H |
| $SO_2$ | $CH_3$ | H | H | H | H | H |
| $SO_2$ | $CH_2Ph$ | H | H | H | H | H |
| $SO_2$ | $CH_2Ph$ | 2-Cl | 3-Cl | H | H | H |
| O | H | H | H | H | H | H |
| O | H | 2-F | 3-F | H | H | H |
| O | H | 2-Cl | 3-Cl | H | H | H |
| O | H | 2-Br | 3-Br | H | H | H |
| O | H | 2-Cl | 3-Cl | 8-$CH_3$ | H | H |
| N-CN | H | H | H | H | H | H |
| N-CN | $CH_3$ | 2-$CH_3$ | 3-$CH_3$ | H | H | H |
| N-CN | $CH_3$ | H | H | H | H | 7-$OCH_3$ |
| S | H | H | H | H | H | 7-$OCH_3$ |
| S | H | H | H | H | 8-Cl | 7-$OCH_3$ |

| Z | $R_1$ | $R_2$ | $R_3$ | $R_4$ | $R_5$ | T |
|---|---|---|---|---|---|---|
| S | $CH_3$ | H | H | H | 8-Cl | 7-$OCH_3$ |
| S | $CH_3$ | H | H | H | 8-Cl | 7-OH |
| S | $CH_3$ | H | H | H | H | 7-$OCH_3$ |
| S | H | H | H | H | H | 7-OH |
| S | $CH_3$ | H | H | H | H | 7-OH |
| SO | H | H | H | H | H | 7-$OCH_3$ |
| SO | $CH_3$ | H | H | H | H | 7-$OCH_3$ |
| S | $SO_2$Ph-p-Me | H | H | H | H | 7-$OCH_3$ |
| $SO_2$ | H | H | H | H | H | 7-$OCH_3$ |
| S | $COCH_3$ | H | H | H | H | 7-$OCH_3$ |
| S | $COCH_3$ | H | H | H | H | H |
| $SO_2$ | H | H | H | H | H | H |
| S | $CH_2CO_2C_2H_5$ | H | H | H | H | H |
| S | $CH_2CO_2H$ | H | H | H | H | H |
| S | $CO_2CH_3$ | H | H | H | H | H |
| S | $CH=CHCO_2CH_3$ | H | H | H | H | H |
| S | H | 2-Cl | H | H | H | H |
| S | H | 3-Cl | H | H | H | H |
| S | H | 8-Cl | H | H | H | H |
| S | H | 2-Me | H | H | H | H |

9. Zusammensetzung nach Anspruch 1, welche zusätzlich einen zweiten wirksamen Bestandteil umfaßt, der ein nicht-steroidales entzündungshemmendes Mittel; ein peripheres Analgetikum; ein Cyclooxygenaseinhibitor; ein Leukotrienantagonist; ein Antihistaminikum; ein Prostaglandinantagonist oder ein Thromboxanantagonist ist.

10. Zusammensetzung nach Anspruch 9, worin der zweite wirksame Bestandteil ein nicht-steroidales entzündungshemmendes Mittel ist.

11. Zusammensetzung nach Anspruch 10, worin das nichtsteroidale entzündungshemmende Mittel Indomethacin ist.

12. Zusammensetzung nach Anspruch 9, worin das Gewichtsverhältnis der Verbindung nach Anspruch 1 zum zweiten wirksamen Bestandteil im Bereich von 1000:1 is 1:1000 liegt.

13. Zusammensetzung nach Anspruch 12, worin das Gewichtsverhältnis im Bereich von 200:1 bis 1:200 liegt.

14. Zusammensetzung nach einem der Ansprüche 1 bis 8 oder Verbindung mit der in Anspruch 1 angegebenen Formel mit den in einem der Ansprüche 1 bis 8 definierten Variablen zur Verwendung bei der therapeutischen Behandlung von Säugetieren, insbesondere von Menschen.

15. Zusammensetzung nach einem der Ansprüche 1 bis 8 oder Verbindung mit der in Anspruch 1 angegebenen Formel mit den in einem der Ansprüche 1 bis 8 definierten Variablen zur Verwendung zur (a) Inhibierung der Leukotrienbiosynthese oder -wirkung in Säugetieren; (b) Behandlung oder Linderung

von Lungenzuständen; (c) Behandlung von Entzündungen; (d) Behandlung von Allergien; oder (e) Behandlung von Hautzuständen.

**16.** Verbindungen mit der Formel I:

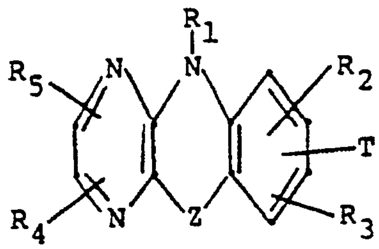

I

worin die Variablen wie folgt sind

| Z | $R_1$ | $R_2$ | $R_3$ | $R_4$ | $R_5$ | T |
|---|---|---|---|---|---|---|
| S | H | H | H | H | H | 7-$OCH_3$ |
| S | H | H | H | H | 8-Cl | 7-$OCH_3$ |
| S | $CH_3$ | H | H | H | 8-Cl | 7-$OCH_3$ |
| S | $CH_3$ | H | H | H | 8-Cl | 7-OH |
| S | $CH_3$ | H | H | H | H | 7-$OCH_3$ |
| S | H | H | H | H | H | 7-OH |
| S | $CH_3$ | H | H | H | H | 7-OH |
| SO | H | H | H | H | H | 7-$OCH_3$ |
| SO | $CH_3$ | H | H | H | H | 7-$OCH_3$ |
| S | $SO_2$Ph-p-Me | H | H | H | H | 7-$OCH_3$ |
| $SO_2$ | H | H | H | H | H | 7-$OCH_3$ |
| S | $COCH_3$ | H | H | H | H | 7-$OCH_3$ |
| S | $COCH_3$ | H | H | H | H | H |
| $SO_2$ | H | H | H | H | H | H |
| S | $CH_2CO_2C_2H_5$ | H | H | H | H | H |
| S | $CH_2CO_2H$ | H | H | H | H | H |
| S | $CO_2CH_3$ | H | H | H | H | H |
| S | $CH=CHCO_2CH_3$ | H | H | H | H | H |

**17.** 7-Methoxy-1,4-diaza-10-methylphenothiazin.

**18.** 7-Hydroxy-1,4-diaza-10H-phenothiazin.

**19.** 7-Hydroxy-1,4-diaza-10-methylphenothiazin.

**20.** 7-Methoxy-1,4-diaza-10-methylphenothiazin.

**21.** 8-Chlor-1,4-diaza-7-methoxyphenothiazin.

**22.** 8-Chlor-7-methoxy-1,4-diaza-10-methylphenothiazin.

**23.** 8-Chlor-7-hydroxy-1,4-diaza-10-methylphenothiazin.